# EUROPÄISCHE PATENTSCHRIFT

(11) **EP 3 218 025 B1**
(45) Veröffentlichungstag und Bekanntmachung des Hinweises auf die Patenterteilung: **24.06.2020**
(21) Anmeldenummer: 15798352.9
(22) Anmeldetag: 10.11.2015
(51) Int. Cl.: A61L 27/36

(54) **HERSTELLUNG UND ISOLIERUNG EINER NATIVEN HUMANEN DERMALEN EXTRAZELLULÄREN MATRIX**
PRODUCTION AND ISOLATION OF A NATIVE HUMAN DERMAL EXTRACELLULAR MATRIX
PRODUCTION ET ISOLEMENT D'UNE MATRICE EXTRACELLULAIRE DERMIQUE HUMAINE NATIVE

(30) Priorität: 10.11.2014 DE 102014222898
(43) Veröffentlichungstag der Anmeldung: 20.09.2017
(73) Patentinhaber: Universität Stuttgart, 70174 Stuttgart (DE); Universität Konstanz, 78464 Konstanz (DE)
(72) Erfinder: RUFF, Mara, 73728 Esslingen (DE); KLUGER, Petra, 70839 Gerlingen (DE); BACH, Monika, 70771 Leinfelden-Echterdingen (DE); TOVAR, Günter, 71111 Waldenbuch (DE); WITTMANN, Valentin, 78465 Konstanz (DE); WIELAND, Daniel, 88400 Biberach an der Riß (DE)
(74) Vertreter: Schrell, Andreas
(86) Internationale Anmeldenummer: PCT/EP2015/076216
(87) Internationale Veröffentlichungsnummer: WO 2016/075141

(56) Entgegenhaltungen:
- WO-A1-2014/039427
- WO-A2-2009/076391
- DE-A1-102011 002 839
- LAUGHLIN S T ET AL: "Metabolic Labeling of Glycans with Azido Sugars for Visualization and Glycoproteomics", METHODS IN ENZYMOLOGY; GLYCOBIOLOGY, ACADEMIC PRESS, US, Bd. 415, 14. November 2006 (2006-11-14), Seiten 230-250, XP008100511, ISSN: 0076-6879, DOI: 10.1016/S0076-6879(06)15015-6 [gefunden am 2006-11-18]
- Anonymous: "Mara Ruff M. Sc.", Mitarbeiterverzeichnis, 1. Januar 2011 (2011-01-01), XP055239261, Gefunden im Internet: URL:http://www.igvp.uni-stuttgart.de/mitar beiter/Ruff_M._Sc./ [gefunden am 2016-01-07]
- Anonymous: "Biologische Beschichtung für Implantate", , 22. Dezember 2014 (2014-12-22), XP055239248, Gefunden im Internet: URL:http://www.igvp.uni-stuttgart.de/news/ Biologische_Beschichtung_fuer_Implantate/? __locale=de [gefunden am 2016-01-07]

## Beschreibung

Die vorliegende Erfindung betrifft funktionalisierte extrazelluläre Matrizes, Verbünde aus funktionalisierten extrazellulären Matrizes und Trägern, Verfahren zu deren Herstellung sowie Verwendungen derselben.

Die extrazelluläre Matrix (im Folgenden EZM genannt), also die in einem biologischen Gewebe die Zellen umgebende und sie enthaltende Interzellularsubstanz, dies beinhaltet auch eine Basallamina, stellt einen wesentlichen Anteil tierischen und humanen Gewebes dar. Sie setzt sich aus verschiedenen Komponenten zusammen, insbesondere Proteinen, Proteoglykanen, Glykoproteinen, Polysacchariden, gegebenenfalls Lipiden sowie Vitaminen, Nährstoffen, Hormonen, Elektrolyten und Wasser. Der dominierende Anteil der Proteine wird durch Kollagene, die insbesondere in Form von Fasern vorliegen, Fibrillin und Elastin gebildet. Ein weiterer großer Anteil der Matrixkomponenten wird durch Kohlenhydrate gebildet, die in erster Linie in Glykosaminoglykan-Form vorliegen und mit Proteinen Proteoglykane oder Glykoproteine bilden. Die genannten Komponenten der EZM werden von Zellen synthetisiert, sezerniert und fixieren sich extrazellulär. EZMs sind an der Rezeptor-vermittelten Expression von Genen, der Zelladhäsion, der Zellmigration, der Zellproliferation sowie dem Aufbau, Umbau und Abbau von Geweben beteiligt und spielen daher eine zentrale Rolle im Metabolismus eines tierischen und humanen Organismus.

Jeder einzelne in einem Organismus vorhandene Zelltyp stellt eine einzigartige, gewebespezifische EZM her, die unterschiedliche biologische Reaktionen induziert. So kann eine EZM je nach Herkunft zum Beispiel entweder die Differenzierung von Stammzellen fördern oder das Stammzellpotenzial erhalten. Dies kann insbesondere für den Einsatz in speziellen Zellkulturen von Interesse sein. Sowohl in Forschung als auch in anwendungsorientierten Technologiefeldern besteht daher ein großes Interesse daran, EZMs an Trägern, beispielsweise Zellkulturschalen oder anderen, insbesondere biokompatiblen Trägern, zu fixieren.

Zellkulturschalen werden standardmäßig aus Polystyrol hergestellt und für ein verbessertes Zellwachstum mit Plasma behandelt (tissue culture polystyrole). Durch die Plasmabeschichtung stehen ausreichend Wasserstoffbrückendonoren und -akzeptoren sowie polare und ionische Gruppen auf der Oberfläche zur Verfügung, sodass dem Medium zugesetzte fötale Serumproteine (fetal calf serum, FCS) adsorbieren können. Während dies für Zellkulturen ausreichend ist, müssen für primäre Zellen in der Regel EZM-Proteine auf der Oberfläche physisorbiert werden, gegebenenfalls unterstützt durch Plasmatechnologie. Zwar sind Verfahren bekannt, um einzelne Proteine gezielt mit funktionellen Gruppen auszustatten und für kovalente Bindungen zu nutzen, diese Methoden sind jedoch sehr aufwändig, müssen für jedes Protein einzeln etabliert werden und sind dadurch mit hohen Kosten verbunden. Für die Beschichtung mit einer komplexen EZM sind diese Methoden daher nicht geeignet. Bisher wird die Beschichtung mit EZM nur über Physisorption durchgeführt. Häufig wird das Substrat dazu zunächst mit Zellen besiedelt, nach entsprechender Kulturdauer werden die Zellen lysiert und die EZM-Beschichtung bleibt als Ablagerung auf dem Substrat zurück. Diese Beschichtung bestätigt die hohe biologische Aktivität der EZM, die Technologie ist jedoch nicht auf jedes Material gleichermaßen übertragbar und mit hohem Aufwand und damit auch hohen Kosten verbunden. Als besonders nachteilig erweist sich, dass eine derartig aufgebrachte EZM auf einem Träger (insbesondere einem planaren und glatten Material) keine große Haltbarkeit aufweist, insbesondere leicht und schnell abgewaschen wird oder sich in anderer Art und Weise löst.

Auch im therapeutisch orientierten Bereich der Entwicklung von Implantaten ist es wünschenswert, mit einer hohen Empfänger-Akzeptanz ausgestattete Implantate bereitzustellen. Häufig sind nämlich Implantate zur Versorgung von Knochen- und Gelenkdefekten aus Metall aufgebaut. Diese stellen für den Organismus einen Fremdkörper dar, dessen ossäre Integration trotz insgesamt guter Bioverträglichkeit immer noch problematisch ist. Zur Verbesserung der Oberflächeneigenschaften von metallischen Implantaten wurden daher verschiedenste Möglichkeiten untersucht, insbesondere auch die Modifikation der Oberflächenstruktur und -eigenschaft von Implantaten, zum Beispiel auch die Ausbildung von Oberflächenmodifikationen an Metallen, die knochenähnliche Strukturen nachbilden sollen. Bekannt ist es auch, Biomaterialien mit Hydroxylapatit-Beschichtungen zu versehen.

Laughlin et al. (Methods in Enzymology, Glycobiology, 2006) offenbaren die Markierung von Glykanen durch metabolisches Oligosaccharid-Engineering, wobei eine Azidgruppe als funktionelle Gruppe kovalent in Glykane eingebaut sein kann. In der WO 2014/039427 A1 wird die Anlagerung von extrazellulärer Matrix auf Oberflächen sowie die nachträgliche chemische Modifikation und Vernetzung einer extrazellulären Matrix offenbart. In der WO 2009/076391 A2 wird eine modifizierte extrazelluläre Matrix, die das Einwachsen von Gewebe fördert, und die nachträgliche Quervernetzung dieser Matrix offenbart. In der DE10 2011 002839 A1 wird ein Verfahren zur Immobilisierung und Aufbereitung funktioneller Multikomponentenstrukturen der extrazellulären Matrix offenbart, wobei eine Haftvermittlerschicht kovalent auf einen Träger gebunden wird und die extrazelluläre Matrix durch Anbindung an die Haftvermittlerschicht immobilisiert wird.

All diesen Verfahren sind jedoch signifikante Nachteile eigen. Bei der Anheftung einzelner Proteine an Träger entstehen hohe, durch eine aufwändige Herstellung, Aufreinigung und Isolierung der einzelnen Proteine ausgelöste Kosten. Hinzu treten mögliche Immunogenitätsprobleme sowie eine häufig auftretende unerwünschte vorzeitige Desorption der Proteine. Eine kovalente Beschichtung von Trägern über in den Proteinen vorhandene OH-, NH₂-, SH- oder COOH-Gruppen führt durch eine veränderte Konformation häufig zum Verlust der biologischen Aktivitäten und ist darüber hinaus unspezifisch.

Der vorliegenden Erfindung liegt daher das technische Problem zu Grunde, Beschichtungen von Trägern, insbesondere biokompatiblen Trägern, mit einer EZM bereitzustellen, die die vorgenannten Nachteile überwinden, insbesondere in einfacher Art und Weise ohne aufwendige Aufreinigungs- und Isolierverfahren herstellbar sind, keine Immunogenität aufweisen, kostengünstig und steril bereitgestellt werden können, und wobei die Beschichtung in Struktur, Physiologie und Funktion einer natürlicherweise vorkommenden EZM gleichkommt.

Die vorliegende Erfindung löst das ihr zu Grunde liegende technische Problem durch die Lehren der unabhängigen Ansprüche.

Insbesondere löst die vorliegende Erfindung das ihr zu Grunde liegende technische Problem durch die Bereitstellung einer funktionalisierten extrazellulären Matrix, im Folgenden FEZM genannt, umfassend Proteine und Kohlenhydrate, die mindestens eine funktionelle Gruppe A aufweist, wobei die mindestens eine funktionelle Gruppe A fähig ist, mindestens eine kovalente Bindung mit einer komplementären funktionellen Gruppe B einzugehen und wobei die funktionelle Gruppe A ausgewählt ist aus der Gruppe bestehend aus einer Azidgruppe, C-C-Doppelbindung und C-C-Dreifachbindung.

Insbesondere löst die vorliegende Erfindung das ihr zu Grunde liegende technische Problem durch die Bereitstellung einer funktionalisierten extrazellulären Matrix, im Folgenden FEZM genannt, umfassend Proteine und Kohlenhydrate, insbesondere umfassend Proteoglykane und Glykoproteine, insbesondere umfassend Oligosaccharidketten aufweisende Proteoglykane und Glykoproteine, wobei die Matrix mindestens eine funktionelle Gruppe A aufweist, wobei die mindestens eine funktionelle Gruppe A kovalent in die Oligosaccharidketten der Proteoglykane oder Glykoproteine eingebaut ist, wobei die mindestens eine funktionelle Gruppe A fähig ist, mindestens eine kovalente Bindung mit einer komplementären funktionellen Gruppe B einzugehen und wobei die funktionelle Gruppe A ausgewählt ist aus der Gruppe bestehend aus einer Azidgruppe, C-C-Doppelbindung und C-C-Dreifachbindung. In einer besonders bevorzugten Ausführungsform können die funktionellen Gruppen A in einer linearen, verzweigten, zyklischen, gesättigten oder ungesättigten Alkylkette oder in einer Arylgruppe vorliegen. In einer Ausführungsform kann eine C-C-Doppelbindung als lineares oder zyklisches Alken vorliegen. In einer Ausführungsform kann eine C-C-Dreifachbindung als lineares oder zyklisches Alkin vorliegen. Erfindungsgemäß umfassen C-N-Dreifachbindungen unter anderem auch Isonitrile und Nitriloxide. In einer besonders bevorzugten Ausführungsform kann die C-C-Doppelbindung als Vinylsulfid ausgeführt sein.

Gemäß der vorliegenden Offenbarung kann die funktionelle Gruppe A ausgewählt sein aus der Gruppe bestehend aus einer Azidgruppe, C-C-Doppelbindung, C-C-Dreifachbindung, C-N-Dreifachbindung, Diengruppe, Imingruppe, Ketongruppe, Tetrazingruppe, Thioketongruppe und Thiolgruppe.

Gemäß der vorliegenden Offenbarung kann die funktionelle Gruppe A ausgewählt sein aus der Gruppe bestehend aus einer Azidgruppe, C-C-Doppelbindung, C-C-Dreifachbindung, C-N-Dreifachbindung, Diengruppe, Imingruppe, Ketongruppe, Tetrazingruppe und Thiolgruppe.

Gemäß der vorliegenden Offenbarung kann die funktionelle Gruppe A ausgewählt sein aus der Gruppe bestehend aus einer Azidgruppe, C-C-Doppelbindung, C-C-Dreifachbindung, C-N-Dreifachbindung, Diengruppe, Imingruppe, Ketongruppe, Tetrazingruppe und Thioketongruppe.

Gemäß der vorliegenden Offenbarung kann die funktionelle Gruppe A ausgewählt sein aus der Gruppe bestehend aus einer Azidgruppe, C-C-Doppelbindung, C-C-Dreifachbindung, C-N-Dreifachbindung, Diengruppe, Imingruppe, Ketongruppe und Tetrazingruppe.

Gemäß der vorliegenden Offenbarung kann die funktionelle Gruppe A ausgewählt sein aus der Gruppe bestehend aus einer Aldehydgruppe, Azidgruppe, C-C-Doppelbindung, C-C-Dreifachbindung, C-N-Dreifachbindung, Diengruppe, Ketongruppe, Tetrazingruppe, Thioketongruppe und Thiolgruppe.

Gemäß der vorliegenden Offenbarung kann die funktionelle Gruppe A ausgewählt sein aus der Gruppe bestehend aus einer Aldehydgruppe, Azidgruppe, C-C-Doppelbindung, C-C-Dreifachbindung, C-N-Dreifachbindung, Diengruppe, Ketongruppe, Tetrazingruppe und Thiolgruppe.

Gemäß der vorliegenden Offenbarung kann die funktionelle Gruppe A ausgewählt sein aus der Gruppe bestehend aus einer Aldehydgruppe, Azidgruppe, C-C-Doppelbindung, C-C-Dreifachbindung, C-N-Dreifachbindung, Diengruppe, Ketongruppe, Tetrazingruppe und Thioketongruppe.

Gemäß der vorliegenden Offenbarung kann die funktionelle Gruppe A ausgewählt sein aus der Gruppe bestehend aus einer Aldehydgruppe, Azidgruppe, C-C-Doppelbindung, C-C-Dreifachbindung, C-N-Dreifachbindung, Diengruppe, Ketongruppe und Tetrazingruppe.

Gemäß der vorliegenden Offenbarung kann die funktionelle Gruppe A ausgewählt sein aus der Gruppe bestehend aus einer Azidgruppe, C-C-Doppelbindung, C-C-Dreifachbindung, C-N-Dreifachbindung, Diengruppe, Ketongruppe, Tetrazingruppe, Thioketongruppe und Thiolgruppe.

Gemäß der vorliegenden Offenbarung kann die funktionelle Gruppe A ausgewählt sein aus der Gruppe bestehend aus einer Azidgruppe, C-C-Doppelbindung, C-C-Dreifachbindung, C-N-Dreifachbindung, Diengruppe, Ketongruppe, Tetrazingruppe und Thiolgruppe.

Gemäß der vorliegenden Offenbarung kann die funktionelle Gruppe A ausgewählt sein aus der Gruppe bestehend aus einer Azidgruppe, C-C-Doppelbindung, C-C-Dreifachbindung, C-N-Dreifachbindung, Diengruppe, Ketongruppe, Tetrazingruppe und Thioketongruppe.

Gemäß der vorliegenden Offenbarung kann die funktionelle Gruppe A ausgewählt sein aus der Gruppe bestehend aus einer Azidgruppe, C-C-Doppelbindung, C-C-Dreifachbindung, C-N-Dreifachbindung, Diengruppe, Ketongruppe und Tetrazingruppe.

Die vorliegende Erfindung stellt demgemäß eine FEZM bereit, die der natürlicherweise von Zellen gebildeten EZM entspricht, vorzugsweise zu ihr identisch ist, mit der Maßgabe, dass sie sich von dieser dadurch entscheidet, dass sie funktionalisiert ist. Diese Funktionalisierung wird durch die in der EZM der vorliegenden Erfindung vorgesehene mindestens eine funktionelle Gruppe A realisiert, welche in natürlicherweise vorkommenden EZMs nicht vorliegt.

Die vorliegende Erfindung stellt daher in vorteilhafter Weise FEZMs bereit, die vorzugsweise natürlicherweise vorkommenden EZMs in Struktur, Physiologie und Funktion entsprechen und insbesondere, mit Ausnahme der mindestens einen funktionellen Gruppe A, identisch zu natürlicherweise vorkommenden EZMs sind und die sich in einfacher Art und Weise, insbesondere ohne aufwendige Aufreinigungs- und Isolierverfahren, insbesondere ohne jegliche Aufreinigungs- und Isolierverfahren, herstellen lassen. Darüber hinaus ist die Bereitstellung der erfindungsgemäßen FEZMs mit lediglich geringen Kosten verbunden und stellt einen großen Vorteil insofern dar, als dass bei Verwendung spendereigener Zellen zur Herstellung der FEZMs bei einer anschließenden Implantation unter Einsatz dieser FEZM keine Immunogenität verursacht wird.

Als erfindungsgemäß besonders vorteilhaft erweist sich, dass die Einführung der mindestens einen funktionellen Gruppe A durch ein chemisches Agens, das als ein eine funktionelle Gruppe A aufweisendes Saccharid ausgeführt ist, in die Glykanstruktur einer FEZM-Komponente, insbesondere eines Proteoglykans oder Glykoproteins, eine Änderung der Konformation und damit der biologischen Aktivität eines Proteins vollständig oder nahezu ausschließt.

Erfindungsgemäße FEZMs lassen sich gewinnen, indem Zellen beliebiger Art, die natürlicherweise zur EZM-Bildung befähigt sind, insbesondere mesenchymale Zellen, insbesondere Epithelzellen, Endothelzellen oder Zellen des Binde- und Stützgewebes bereitgestellt und in Zellkultur mit mindestens einem chemischen Agens mit einer funktionellen Gruppe A ausgewählt aus der Gruppe bestehend aus einer Azidgruppe, C-C-Doppelbindung und C-C-Dreifachbindung, kultiviert werden und während der Kultivierung Bestandteile einer EZM bilden und sezernieren, die sich durch die Anwesenheit der durch das mindestens eine chemische Agens in die EZM eingeführten funktionellen Gruppe A auszeichnet. Die sezernierten FEZM-Bestandteile mit der mindestens einen funktionellen Gruppe A fixieren sich außerhalb der Zelle zu einer FEZM. Die von diesen so kultivierten Zellen gebildete FEZM stellt daher eine von Zellen in vitro hergestellte, mit mindestens einer funktionellen Gruppe A modifizierte EZM dar.

Die genannte Herstellung der FEZM wird im Folgenden auch als metabolisches Oligosaccharid-Engineering bezeichnet und stellt ein Verfahren dar, mindestens eine Substanz, insbesondere ein chemisches Agens mit mindestens einer funktionellen Gruppe A in lebenden Zellen in Bestandteile einer EZM einzubauen und diese so funktionalisierten Bestandteile im Anschluss aus der Zelle zu sezernieren. Für dieses Verfahren zur Herstellung einer FEZM können neben Fibroblasten auch andere primäre Zellen humanen oder tierischen Ursprungs eingesetzt werden, zum Beispiel Adipozyten, Chondrozyten, Endothelzellen, Epithelzellen, Hepatozyten, Keratinozyten, adulte Stammzellen oder embryonale Stammzellen (insbesondere mesenchymale Stammzellen), Osteoblasten, Perizyten sowie Zellen des Binde- und Stützgewebes.

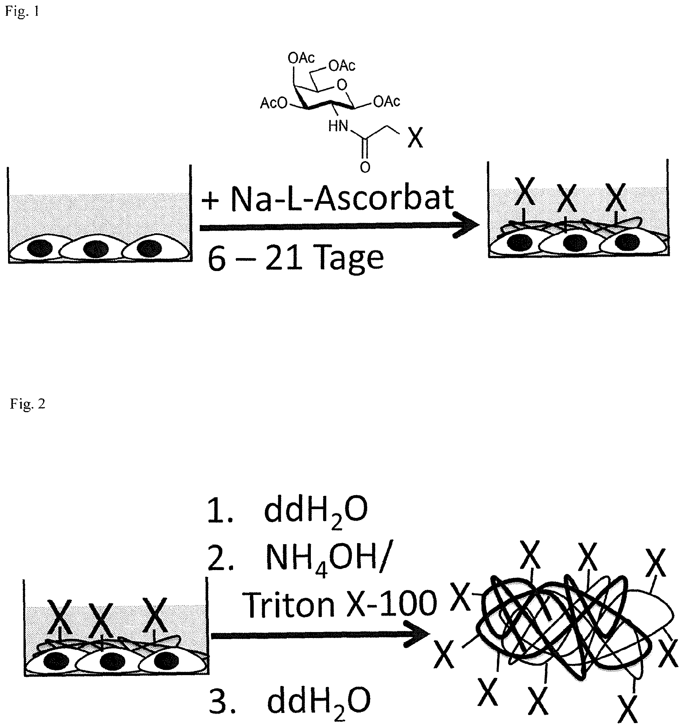

Im Zusammenhang mit der vorliegenden Erfindung wird unter einem metabolischen Oligosaccharid-Engineering ein Verfahren verstanden, gemäß dem Zellen eines bestimmten oder mehrerer Zelltypen unter Zugabe mindestens eines chemischen Agens, insbesondere eines Saccharidderivates, kultiviert werden, wobei das chemische Agens mit einer funktionellen Gruppe A ausgewählt aus der Gruppe bestehend aus einer Aldehydgruppe, Azidgruppe, C-C-Doppelbindung, C-C-Dreifachbindung, C-N-Dreifachbindung, Diengruppe, Imingruppe, Ketongruppe, Tetrazingruppe, Thioketongruppe und Thiolgruppe in die Zelle eindringt und dort unter Nutzung des Zellmetabolismus in gebildete Makromoleküle, insbesondere Proteoglykane und Glykoproteine, insbesondere in deren Oligosaccharidketten, so, insbesondere kovalent, eingebaut wird, dass zumindest eine funktionelle Gruppe A des chemischen Agens in dem gebildeten Makromolekül verbleibt und so frei für eine kovalente Bindung mit einer funktionellen Gruppe B vorliegt. Die Erfindung macht sich dabei an sich bekannte Verfahren der bioorthogonalen Ligation zunutze, insbesondere sieht die erfindungsgemäße Verfahrensweise vor, dass der Einbau der funktionellen Gruppe A in das gebildete Makromolekül ohne Schädigung, Beeinträchtigung oder Störung der biologischen Funktion der Zelle erfolgt. Das so gebildete funktionalisierte, mindestens eine funktionelle Gruppe A aufweisende Makromolekül wird von der Zelle anschließend sezerniert und bildet einen integralen Bestandteil der sich extrazellulär formierenden FEZM. Schematisch wird das metabolische Oligosaccharid-Engineering in Figur 1 dargestellt. Insbesondere wird die durch das mindestens eine chemische Agens eingeführte funktionelle Gruppe A in ein Proteoglykan oder ein Glykoprotein, insbesondere ein Glykosaminoglykan eingebaut, insbesondere kovalent in ein Proteoglykan oder Glykoprotein eingebaut, insbesondere in die Glykanstruktur eines Proteoglykans oder Glykoproteins.

Im Zusammenhang mit der vorliegenden Erfindung wird unter einer EZM eine von Zellen eines bestimmten Typs gebildete und extrazellulär abgeschiedene Interzellularsubstanz verstanden, die im in vivo-Zustand den Intrazellularraum ausfüllt und üblicherweise insbesondere aus Proteinen, Kohlenhydraten, Lipiden, Hormonen, Vitaminen und gegebenenfalls anorganischen Bestandteilen aufgebaut ist. In jedem Fall weisen EZMs Proteine und Kohlenhydrate auf, wobei diese insbesondere auch in Mischform vorliegen, das heißt als Proteoglykane und Glykoproteine, insbesondere als Glykosaminoglykane. Eine EZM im Sinne der vorliegenden Erfindung ist daher in besonders bevorzugter Ausführungsform eine von einem bestimmten Zelltyp gebildete und sezernierte Interzellularsubstanz, umfassend zumindest Proteine und Kohlenhydrate, insbesondere Proteoglykane und Glykoproteine. Besonders bevorzugt ist eine EZM, die einer natürlicherweise vorkommenden EZM eines bestimmten Zelltyps vollständig oder nahezu vollständig entspricht, das heißt, die gleiche Struktur, Physiologie und Funktion wie eine natürlicherweise vorkommende EZM aufweist.

Im Zusammenhang mit der vorliegenden Erfindung wird unter einer EZM auch eine Basalmembran, insbesondere eine Basallamina, verstanden. Synthetisch nicht von Zellen hergestellte EZMs, die der Struktur, Physiologie und Funktion von durch lebende Zellen gebildeten EZM entsprechen, werden durch die vorliegende Erfindung ebenfalls erfasst.

Im Zusammenhang mit der vorliegenden Erfindung wird unter einer FEZM eine EZM verstanden, die mindestens eine funktionelle Gruppe A aufweist, die fähig ist, mindestens eine, bevorzugt mindestens zwei, kovalente Bindung(en) mit einer komplementären Gruppe B zu bilden. Die FEZM der vorliegenden Erfindung wird bevorzugt durch metabolisches Oligosaccharid-Engineering hergestellt.

Im Zusammenhang mit der vorliegenden Erfindung wird unter der funktionellen Gruppe A eine erste funktionelle Gruppe verstanden, die fähig ist, mit einer zweiten funktionellen Gruppe B mindestens eine kovalente Bindung einzugehen, wobei die funktionelle Gruppe A ausgewählt ist aus der Gruppe bestehend aus einer Aldehydgruppe, Azidgruppe, C-C-Doppelbindung, C-C-Dreifachbindung, C-N-Dreifachbindung, Diengruppe, Imingruppe, Ketongruppe, Tetrazingruppe, Thioketongruppe und Thiolgruppe. Durch die Auswahl der funktionellen Gruppe A wird es möglich, die gebildete FEZM ortsspezifisch auf der Oberfläche eines Trägers zu immobilisieren, welcher eine funktionelle Gruppe B aufweist. Zudem können die funktionellen Gruppen genutzt werden, weitere Biomoleküle wie Wachstumsfaktoren, Inhibitoren oder Signalmoleküle gezielt an der EZM zu immobilisieren.

Im Zusammenhang mit der vorliegenden Erfindung wird unter der funktionellen Gruppe B eine zweite funktionelle Gruppe verstanden, die fähig ist, mit einer ersten funktionellen Gruppe A mindestens eine kovalente Bindung einzugehen, wobei die funktionelle Gruppe B ausgewählt ist aus der Gruppe bestehend aus einer Aminooxygruppe, Azidgruppe, C-C-Doppelbindung, C-C-Dreifachbindung, O-Chinonmethid, C-N-Dreifachbindung, Cystamingruppe, Diengruppe, Hydrazidgruppe, Iodphenylgruppe, Nitrilimingruppe, Nitriloxidgruppe, Phosphingruppe, Phosphitgruppe, Tetrazingruppe, Thiocarbonsäuregruppe und Thiolgruppe. In einer besonders bevorzugten Ausführungsform können die funktionellen Gruppen B in einer linearen, verzweigten, zyklischen, gesättigten oder ungesättigten Alkylkette oder in einer Arylgruppe vorliegen. In einer Ausführungsform kann eine C-C-Doppelbindung als lineares oder zyklisches Alken vorliegen. In einer Ausführungsform kann eine C-C-Dreifachbindung als lineares oder zyklisches Alkin vorliegen. Erfindungsgemäß umfassen C-N-Dreifachbindungen unter anderem auch Isonitrile und Nitriloxide. In einer besonders bevorzugten Ausführungsform kann das zyklische Alkin Cyclooctin oder ein Derivat davon, insbesondere Dibenzocyclooctin, sein.

Erfindungsgemäß wird unter einem Derivat des Cyclooctins eine Verbindung verstanden, die an mindestens einer Stelle eines H-Atoms ein anderes Atom oder Atomgruppe besitzt oder bei der an mindestens einer Stelle ein Atom entfernt wurde.

In besonders bevorzugter Ausfiihrungsform ist die funktionelle Gruppe B eine Nitrilimingruppe, die photochemisch aus einem Tetrazol erzeugt wird und mit einem linearen oder zyklischen Alken in einer Photoklick-Reaktion reagieren kann.

Erfindungsgemäß werden unter einer Phosphingruppe Verbindungen aus einem dreiwertigen Phosphoratom verstanden, an die Wasserstoff oder organische Substitutenten gebunden sind, die mit Aziden insbesondere in einer Staudinger-Ligation reagieren können. Erfindungsgemäß werden unter einer Phosphitgruppe Ester der Phosphonsäure der Form P(OR)₃ mit R als Wasserstoff, Alkyl- oder Aryl-Rest verstanden, die mit Aziden insbesondere in einer Staudinger-Phosphit-Ligation reagieren können.

Im Zusammenhang mit der vorliegenden Erfindung werden die funktionellen Gruppen A und B, die fähig sind, miteinander mindestens eine kovalente Bindung einzugehen, als zueinander komplementäre Gruppen bezeichnet. Eine zu der funktionellen Gruppe A komplementäre Gruppe ist also die funktionelle Gruppe B. Eine zu der funktionellen Gruppe B komplementäre Gruppe ist also die funktionelle Gruppe A.

Erfindungsgemäß wird unter einem chemischen Agens mit einer funktionellen Gruppe A ausgewählt aus der Gruppe bestehend aus einer Azidgruppe, C-C-Doppelbindung und C-C-Dreifachbindung ein Saccharid, insbesondere ein Mono- oder Disaccharid, vorzugsweise ein Monosaccharid, mit einer funktionellen Gruppe A verstanden, das heißt ein Saccharidderivat. Insbesondere liegt die funktionelle Gruppe A nach der kovalenten Bindung des Saccharidderivates an eine Komponente der EZM, insbesondere ein Proteoglykan oder Glykoprotein, frei vor und kann mit einer komplementären funktionellen Gruppe B eines anderen Moleküls, insbesondere eines geträgerten Moleküls, mindestens eine kovalente Bindung eingehen. In besonders bevorzugter Ausführungsform ist das Saccharid, insbesondere das Mono- oder Disaccharid, mit der funktionellen Gruppe A ein peracyliertes Saccharid, insbesondere ein peracetyliertes Saccharid, mit einer funktionellen Gruppe A. In besonders bevorzugter Ausführungsform ist das Mono- oder Disaccharid ein *N*-Acetylgalactosamin oder ein *N-*Acetylmannosamin mit einer funktionellen Gruppe A.

In besonders bevorzugter Ausführungsform wird ein Azid-modifiziertes Mono- oder Disaccharid eingesetzt. In besonders bevorzugter Ausführungsform wird ein Azid-, Alken- oder Alkin-modifiziertes Monosaccharid eingesetzt, wie *N*-Azidoacetylgalactosamin, *N*-Alkenylacetylgalactosamin, *N-*Alkinylacetylgalactosamin, *N*-Azidoacetylmannosamin, *N*-Alkenylacetylmannosamin oder *N-*Alkinylacetylmannosamin. In besonders bevorzugter Ausführungsform wird ein Cyclopropenmodifiziertes Monosaccharid eingesetzt. In besonders bevorzugter Ausführungsform wird ein Carbamatmodifiziertes Monosaccharid eingesetzt. In besonders bevorzugter Ausführungsform wird eine FEZM bereitgestellt, die zusätzlich Biomoleküle, also biologisch oder pharmazeutisch, insbesondere therapeutisch, aktive funktionelle Moleküle enthält, insbesondere kovalent gebunden an eine funktionelle Gruppe A der FEZM.

Die vorliegende Erfindung stellt daher auch Verfahren zur Herstellung, insbesondere zur in vitro-Herstellung, einer FEZM bereit, umfassend die folgenden Verfahrensschritte:
a) Bereitstellen von Zellen,
b) Behandeln und Kultivieren der Zellen mit mindestens einem chemischen Agens enthaltend eine funktionelle Gruppe A ausgewählt aus der Gruppe bestehend aus einer Azidgruppe, C-C-Doppelbindung und C-C-Dreifachbindung unter Bedingungen, dass eine FEZM erzeugt wird, und
c) Gewinnen der FEZM.

In besonders bevorzugter Ausführungsform werden die Zellen in Verfahrensschritt a) in einem Kulturmedium insbesondere einem flüssigen Kulturmedium, üblicher Art unter üblichen Bedingungen bereitgestellt.

Die Bedingungen zur Behandlung und Kultivierung von Zellen gemäß Verfahrensschritt b), die die Erzeugung einer FEZM ermöglichen, sind dem Fachmann geläufige, übliche Kultivierungsbedingungen unter Verwendung üblicher Kultivierungsmittel und -medien, die die Zellen in die Lage versetzen, einerseits ihren Stoffwechsel aufrechtzuerhalten und dabei eine EZM, insbesondere unter Verwendung des zugesetzten chemischen Agens eine FEZM, zu bilden, das heißt das zugesetzte chemische Agens intrazellulär in Proteoglykane oder Glykoproteine von EZM-Bestandteilen einzubauen, diese so modifizierten EZM-Bestandteile aus der Zelle auszuschleusen und wobei die Kulturbedingungen eine FEZM-Bildung außerhalb der Zelle ermöglichen.

In besonders bevorzugter Ausführungsform der vorliegenden Erfindung wird in Verfahrensschritt b) Natrium-L-ascorbat hinzugegeben, vorzugsweise um die Synthese und Sekretion von extrazellulären Matrix-Molekülen zu fördern.

In besonders bevorzugter Ausführungsform ist die bereitgestellte erfindungsgemäße FEZM zellfrei. In besonders bevorzugter Ausführungsform wird eine zellfreie FEZM dadurch bereitgestellt, dass nach Verfahrensschritt b) in einem Verfahrensschritt b') die Zellen, insbesondere durch Lyse, entfernt, werden. In besonders bevorzugter Ausführungsform kann die Entfernung der Zellen durch Zugabe von Ammoniumhydroxidlösung durchgeführt werden. In besonders bevorzugter Ausführungsform kann auch vorgesehen sein, den Verfahrensschritt b') nach Verfahrensschritt c) durchzuführen.

In besonders bevorzugter Ausführungsform findet Verfahrensschritt b') vor Verfahrensschritt c) statt.

In Verfahrensschritt c) wird die FEZM aus dem Kulturmedium gewonnen, insbesondere das Kulturmedium abgetrennt und die Zellen lysiert.

In besonders bevorzugter Ausführungsform kann in Verfahrensschritt c) eine Reinigung der FEZM durch Waschen, insbesondere mit Wasser, erfolgen.

Insbesondere sind die erfindungsgemäß eingesetzten Zellen humane oder tierische Zellen aus einer Primärkultur oder Zellkultur. In besonders bevorzugter Ausführungsform sind die eingesetzten Zellen Epithelzellen, Endothelzellen oder Zellen des Stütz- und Bindegewebes, insbesondere Fibroblasten, adulte oder embryonale Stammzellen (insbesondere mesenchymale Stammzellen), Keratinozyten, Osteoblasten, Osteozyten, Adipozyten, Chondrozyten, Hepatozyten,

In besonders bevorzugter Ausführungsform sind die Zellen autologe Zellen. Im Zusammenhang mit der vorliegenden Erfindung werden unter autologen Zellen solche Zellen verstanden, die aus einem Spenderorganismus stammen, in den die von den erfindungsgemäß eingesetzten Zellen hergestellte FEZM oder ein FEZM-Träger-Verbund der vorliegenden Erfindung, zum Beispiel im Rahmen eines therapeutischen Verfahrens, eingeführt werden.

Die vorliegende Erfindung betrifft auch mittels des erfindungsgemäßen Verfahrens zur Herstellung von FEZMs herstellbare, insbesondere hergestellte, FEZMs.

Eine erfindungsgemäße FEZM kann in besonders bevorzugter Ausführungsform der vorliegenden Erfindung mit einem funktionalisierten Träger verbunden, insbesondere kovalent verbunden, werden.

Ein erfindungsgemäß funktionalisierter Träger weist auf seiner Oberfläche mindestens eine funktionelle Gruppe B auf, die fähig ist, mit mindestens einer funktionellen Gruppe A der FEZM mindestens eine kovalente Bindung auszubilden, wobei die funktionelle Gruppe B ausgewählt ist aus der Gruppe bestehend aus einer Azidgruppe, C-C-Doppelbindung, C-C-Dreifachbindung, Nitrilimingruppe, Nitriloxidgruppe, Phosphingruppe, Phosphitgruppe und Tetrazingruppe. In besonders bevorzugter Ausführungsform weist der funktionalisierte Träger Cyclooctin oder ein Derivat davon als funktionelle Gruppe B auf. In besonders bevorzugter Ausführungsform können Cyclooctin oder seine Derivate in einer linearen, verzweigten oder zyklischen Verbindung vorliegen, die weitere Substituenten und/oder Heteroatome im Ring enthalten kann.

In besonders bevorzugter Ausführungsform ist ein derartiger funktionalisierter Träger ein im biotechnologischen, pharmazeutischen, diagnostischen, therapeutischen oder Zellkultur-bezogenen Bereich Anwendung findender Träger, beispielsweise ein Zellkulturgefäß, eine Prothese oder ein Implantat. Als Materialien eines funktionalisierten Trägers eignen sich insbesondere Kunststoffe, zum Beispiel Polystyrol, Glas, Keramik, Silizium, Kohlenstoff, ein Metall, insbesondere Titan, oder ein Verbundwerkstoff.

Demgemäß können erfindungsgemäße FEZMs kovalent an Oberflächen eines Trägers gebunden werden, welcher mit einer komplementären funktionellen Gruppe B modifiziert ist und so einen funktionalisierten Träger bereitstellt.

Die vorliegende Erfindung betrifft auch einen Kit enthaltend eine erfindungsgemäße FEZM und einen funktionalisierten Träger der vorliegenden Erfindung, optional zusammen mit mindestens einer Reaktionslösung und/oder Instruktionen zur Herstellung eines FEZM-Träger-Verbundes aus der erfindungsgemäßen FEZM und einem funktionalisierten Träger.

Die vorliegende Erfindung stellt auch einen FEZM-Träger-Verbund bereit, umfassend eine erfindungsgemäße FEZM und einen erfindungsgemäß funktionalisierten Träger, wobei die FEZM durch mindestens eine von der mindestens einen funktionellen Gruppe A mit der mindestens einen komplementären funktionellen Gruppe B gebildete kovalente Bindung an die Oberfläche des Trägers gebunden ist, wobei gilt:
i) wenn die funktionelle Gruppe A eine Azidgruppe ist, ist die funktionelle Gruppe B eine C-C-Doppelbindung, C-C-Dreifachbindung, Phosphingruppe oder Phosphitgruppe ,
ii) wenn die funktionelle Gruppe A eine C-C-Doppelbindung ist, ist die funktionelle Gruppe B eine Azidgruppe, C-C-Doppelbindung, C-C-Dreifachbindung, Nitrilimingruppe, Nitriloxidgruppe oder Tetrazingruppe, und
iii) wenn die funktionelle Gruppe A eine C-C-Dreifachbindung ist, ist die funktionelle Gruppe B eine Azidgruppe, C-C-Doppelbindung, Nitriloxidgruppe oder Tetrazingruppe.

Bei der Verwendung von Azid-, Alken- oder Alkin-modifizierten Sacchariden als chemisches Agens, insbesondere Monosacchariden, ist eine erfindungsgemäße Beschichtung eines Trägers möglich, die sich die Azid-Alkin-Cycloaddition, Staudinger-Ligation, Staudinger-Phosphit-Ligation oder Diels-Alder-Reaktion zunutze macht. Alkene können durch eine Diels-Alder-Reaktion kovalent an ein Dien knüpfen. Azide können in einer Staudinger-Ligation kovalent an Phosphine oder Phosphite knüpfen. Azide können in einer Cycloaddition an Alkine knüpfen. Tetrazine können in einer [4+1]-Cycloaddition an Isonitrile knüpfen und in einer Diels-Alder-Reaktion an Alkene und Alkine knüpfen. Alkine können in einer Sonogashira-Kupplung an Iodphenylgruppe knüpfen.

Die vorliegende Erfindung stellt daher einen FEZM-Träger-Verbund bereit, umfassend eine erfindungsgemäße FEZM und einen erfindungsgemäßen funktionalisierten Träger, wobei die mindestens eine funktionelle Gruppe A der FEZM kovalent mit der mindestens einen funktionellen Gruppe B des funktionalisierten Trägers verbunden und so die FEZM kovalent an der Oberfläche des Trägers gebunden ist.

In besonders bevorzugter Ausführungsform stellt die vorliegende Erfindung einen FEZM-Träger-Verbund bereit, wobei die mindestens eine kovalente Bindung zwischen einer Azidgruppe als funktionelle Gruppe A mit Cyclooctin oder einem Derivat davon als funktionelle Gruppe B gebildet ist.

Die erfindungsgemäß bereitgestellte FEZM kann demgemäß kovalent an einen entsprechenden funktionalisierten Träger gebunden werden und so einen FEZM-Träger-Verbund bereitstellen, der sich demgemäß durch eine nahezu einer natürlicherweise vorkommenden EZM gleichkommende FEZM-Beschichtung auszeichnet. Erfindungsgemäße FEZM-Träger-Verbunde können in vielfältiger Weise eingesetzt werden, beispielsweise in therapeutischen Anwendungsfeldern wie der Bereitstellung von FEZM-beschichteten Prothesen oder Implantaten, in der Diagnose oder in Zellkulturtechnologien, zum Beispiel zur Kultivierung von Zellen.

Die erfindungsgemäß ermöglichte kovalente Beschichtung der FEZMs auf Trägern stellt einen signifikanten Vorteil insofern dar, als dass die FEZM-Beschichtung keiner Desorption, das heißt Ablösung von einem Träger, unterliegt.

Da die erfindungsgemäß vorgesehene Beschichtung eines Trägers mit FEZMs eine Makromolekülbasierte Beschichtung ist, kann eine Maskierung, insbesondere zum Beispiel durch Serumproteine, ausgeschlossen werden. Vorteilhafterweise kann die erfindungsgemäß bereitgestellte FEZM unter sterilen Bedingungen hergestellt werden.

So können erfindungsgemäß bevorzugt mit erfindungsgemäßen FEZM beschichtete funktionalisierte Polystyrolschälchen hergestellt und zum Beispiel Einsatz in der speziellen Zellkultur finden. Polystyrol lässt sich mittels Plasmatechnologie und anschließend nasschemischen Verfahren mit funktionellen Gruppen ausstatten. Der FEZM-Träger-Verbund fördert das Anwachsen von frisch isolierten primären humanen Zellen und kann die Dedifferenzierung der Zellen verhindern.

Eine Implantats- oder Prothesenbeschichtung mit einer erfindungsgemäßen FEZM ist für das Einwachsen des Implantats und die Wundheilung von Vorteil. Für den Einsatz als Implantatbeschichtung werden Biomaterialien, insbesondere Titan, bevorzugt. Titan kann mittels Plasmatechnologie oder rein nasschemisch mit funktionellen Gruppen modifiziert und anschließend mit einer FEZM beschichtet werden. Eine so beschichtete Titanoberfläche beschleunigt das Einwachsen des Implantats und bewirkt eine bessere Langzeitstabilität.

Die vorliegende Erfindung betrifft auch die Verwendung einer erfindungsgemäßen FEZM oder eines erfindungsgemäßen FEZM-Träger-Verbundes zur Modifizierung der Oberfläche von Biomaterialien, Medizinprodukten und Zellkultursubstraten, insbesondere zur Verbesserung der Anheftung, der Proliferation, des Wachstums, der Differenzierung oder der Dedifferenzierung von Zellen.

Derartige Zellen können Säugerzellen, insbesondere humane Zellen, zum Beispiel HaCaT-Zellen sein. Die vorliegende Erfindung betrifft daher auch mit Zellen besiedelte FEZMs oder FEZM-Träger-Verbünde.

Erfindungsgemäß hergestellte FEZMs, die mindestens eine funktionelle Gruppe A aufweisen, erweisen sich als besonders vorteilhaft insofern, als dass diese zum Beispiel zur Beschichtung von Trägern, welche eine zu der funktionellen Gruppe A komplementäre Gruppe B aufweisen, sodass durch mindestens eine kovalente Bindung zwischen den beiden funktionellen Gruppen A und B eine stabile FEZM-Beschichtung des Trägers erreicht werden kann.

In besonders bevorzugter Form nutzt die vorliegende Erfindung ein bioorthogonales Ligationsverfahren, um mit einer FEZM beschichtete Träger zu erhalten. Insbesondere wird in einem ersten Schritt ein in einer EZM vorhandenes Substrat, bevorzugt ein Protein, ein Glykoprotein oder ein Proteoglykan, mit einer funktionellen Gruppe A in einer lebenden Zelle modifiziert, wobei die durch die bioorthogonale Ligation eingeführte funktionelle Gruppe A die Zelle in ihrer biologischen Funktion nicht beeinträchtigt und nach der Sekretion aus der Zelle weiterhin unverändert vorliegt. In einem zweiten Schritt erfolgt die kovalente Kopplung der mit der funktionellen Gruppe A versehenen FEZM mit einer für die bioorthogonale Ligation notwendigen komplementären funktionellen Gruppe B.

Die vorliegende Erfindung stellt auch ein Verfahren zur Herstellung eines FEZM-Träger-Verbunds bereit, wobei eine FEZM mit mindestens einer funktionellen Gruppe A mit einem funktionalisierten Träger mit mindestens einer funktionellen Gruppe B unter Bedingungen umgesetzt, insbesondere in Kontakt gebracht, wird, dass die FEZM über mindestens eine kovalente Bindung zwischen der funktionellen Gruppe A und der funktionellen Gruppe B auf dem funktionalisierten Träger, insbesondere an dessen Oberfläche, gebunden wird.

Erfindungsgemäß kann die für die Umsetzung mit dem Träger vorgesehene FEZM eine native oder homogenisierte FEZM sein.

In besonders bevorzugter Ausführungsform wird die FEZM in homogenisierter Form mit dem funktionalisierten Träger in Kontakt gebracht. In besonders bevorzugter Ausführungsform werden daher die durch die Homogenisierung erhaltenen Bestandteile, das heißt Einheiten, der FEZM, die sich jeweils durch die Anwesenheit einer funktionellen Gruppe A auszeichnen, mit dem funktionalisierten Träger in Kontakt gebracht und so mit der funktionellen Gruppe B auf dem funktionalisierten Träger gebunden.

Im Zusammenhang mit der vorliegenden Erfindung wird unter einer in homogenisierter Form vorliegenden FEZM eine FEZM verstanden, die mittels eines Homogenisierungsprozesses unter Verwendung eines Homogenisierungsmittels in gleichförmige kleinere Einheiten, insbesondere in eine Suspension, überführt wurde und so eine homogenisierte FEZM-Suspension bildet. Eine in homogenisierter Form vorliegende FEZM liegt daher nicht mehr als ein fester Makromolekülverbund vor, sondern befindet sich in desintegrierter Form, bevorzugt in Form von vielen kleinen Einheiten in einer Suspension, welche makroskopisch, das heißt mit bloßem Auge betrachtet, einer Lösung gleichkommt. Dabei wird die FEZM und insbesondere auch größere Matrixkomplexe der FEZM aufgebrochen, vorzugsweise ohne kovalente Bindungen zu spalten.

In besonders bevorzugter Ausführungsform liegt die FEZM in einem Homogenisierungsmittel in homogenisierter Form vor. Das Homogenisierungsmittel kann in besonders bevorzugter Ausführungsform eine Säure, insbesondere eine schwache Säure, vorzugsweise Essigsäure, sein. In einer besonders bevorzugten Ausführungsform ist das Homogenisierungsmittel eine 0,05 bis 0,5%ige, insbesondere 0,1%ige, Essigsäure.

In einer besonders bevorzugten Ausführungsform wird die erfindungsgemäß vorliegende FEZM daher bevor sie mit dem funktionalisierten Träger in Kontakt gebracht wird, in einem Homogenisierungsmittel homogenisiert, insbesondere durch sukzessive Zugabe des Homogenisierungsmittels.

In besonders bevorzugter Ausführungsform wird die Homogenisierung durch sukzessive und langsame Zugabe des vorzugsweise sauren Homogenisierungsmittels hergestellt. In besonders bevorzugter Ausführungsform wird in einem ersten Schritt das Homogenisierungsmittel bereitgestellt und anschließend die FEZM, insbesondere enthalten in einem wässrigen Medium, hinzugegeben. In weiteren optionalen Verfahrensschritten werden zu dem so bereitgestellten Gemisch aus Homogenisierungsmittel und FEZM alternierend Homogenisierungsmittel und weitere FEZM hinzugegeben.

In besonders bevorzugter Ausführungsform wird daher eine Homogenisierung der FEZM in einem Homogenisierungsmittel dadurch erreicht, dass alternierend Homogenisierungsmittel und FEZM zu einem Gemisch von FEZM und Homogenisierungsmittel hinzugegeben werden.

In besonders bevorzugter Ausführungsform wird die FEZM vor dem Inkontaktbringen mit dem funktionalisierten Träger in Säure, insbesondere Essigsäure homogenisiert.

In besonders bevorzugter Ausführungsform wird die homogenisierte FEZM vor der Beschichtung des Trägers neutralisiert, bevorzugt durch Zugabe eines Puffers, zum Beispiel von Hepes Puffer. Der für die Neutralisierung eingesetzte Puffer kann in bevorzugter Ausführungsform in einer Konzentration von 1 mM bis 1 M, vorzugsweise 50 mM bis 500 mM, insbesondere 100 mM eingesetzt werden.

Weitere vorteilhafte Ausgestaltungen der Erfindung ergeben sich aus den Unteransprüchen.

Die nachfolgenden Beispiele erläutern die vorliegende Erfindung ohne sie jedoch einzuschränken.

Die Figuren zeigen:
- Figur 1:: eine schematische Darstellung des metabolischen Oligosaccharid-Engineerings,
- Figur 2:: eine schematische Darstellung des Versuchsaufbaus zur Isolierung der von Fibroblasten gebildeten EZMs,
- Figur 3:: eine EZM aus humanen dermalen Fibroblasten nach Behandlung mit einem chemischen Agens,
- Figur 4:: lichtmikroskopische Aufnahmen von FEZMs aus dermalen Fibroblasten,
- Figur 5:: immunhistochemische Färbungen an Paraffin-Dünnschnitten der FEZM aus dermalen Fibroblasten,
- Figur 6:: ein Reaktionsschema zur Beschichtung von Substraten mit FEZM,
- Figur 7:: die Zelladhäsion epidermaler Keratinozyten auf EZM-beschichteten Substraten,
- Figur 8:: die Adhäsion epidermaler Keratinozyten auf FEZM-beschichteten Nexterion®-Slide H-Substraten,
- Figur 9:: fluoreszenzmikroskopischer Nachweis der DIBO-Funktionalisierung,
- Figur 10:: einen Nachweis der FEZM-Beschichtung auf DIBO-funktionalisiserten Siliziumwafern durch Färbung mit Fluorescamin im Vergleich mit einer EZM-Beschichtung,
- Figur 11:: die Adhäsion von primären epidermalen Keratinozyten auf FEZM-beschichteten Titansubstraten,
- Figur 12:: eine schematische Darstellung einer erfindungsgemäßen Prothese,
- Figur 13:: die Stabilität der Beschichtung mit EZM und FEZM, und
- Figur 14:: das Zellwachstum von HaCaT-Zellen auf FEZM-beschichteten Glassubstraten.

### Beispiele:

### Beispiel 1

### Herstellung und Isolierung einer nicht-modifizierten EZM

Aus Haut-Biopsaten können spendereigene dermale Fibroblasten isoliert werden, welche auch in Zellkultur glykosylierte, extrazelluläre Matrixproteine synthetisieren und sezernieren. In etwa sechs bis 21 Tagen entsteht dabei ein dichtes Netzwerk aus Fibroblasten und EZM. Die nicht-modifizierte EZM kann gemäß den folgenden Verfahrensschritten hergestellt werden:
- Aussaat der Fibroblasten in einer Dichte von 20.000 Zellen /cm²
- Kultivierung bei 37 °C, 5 % CO₂ über 6 Tage
- Konditionierter Mediumwechsel an Tag 2 und Tag 5, d.h. die Hälfte des alten Mediums wird entfernt und durch frisches Medium ersetzt
- Zugabe von Na-L-Ascorbat, 50 µg/ml an Tag 1, Tag 2, Tag 5
- Lyse der Zellen an Tag 6 durch Waschen mit dest. Wasser und Inkubation in 20 mM NH₄OH zur Gewinnung der EZM
- Aufreinigung der EZM durch Waschen mit dest. Wasser

Durch die Inkubation mit Ammoniumhydroxid-Lösung wird die Zellmembran der Zellen zerstört, das Zelllysat kann anschließend mit dest. Wasser abgewaschen werden. Die verbleibende EZM ist nicht wasserlöslich, sie bleibt als EZM aus glykosylierten Proteinen und Proteoglykanen erhalten.

### Beispiel 2

### Herstellung, Isolierung und Charakterisierung einer FEZM

### Metabolisches Oligosaccharid-Engineering

Werden die Fibroblasten in Zellkultur mit peracetylierten und funktionalisierten Monosacchariden, z. B. peracetyliertem N-Azidoacetylgalactosamin (Ac₄GalNAz) oder peracetyliertem N-Azidoacetylmannosamin (Ac₄ManNAz) (Fig. 1, X = Azid als funktionelle Gruppe A), behandelt, so werden diese in die Glykan-Strukturen der extrazellulären Matrixproteine eingebaut. Durch Zugabe von Natrium-L-ascorbat wird die Synthese und Sekretion von extrazellulären Matrixmolekülen gefördert. Die FEZM kann gemäß den folgenden Verfahrensschritten hergestellt werden:
- Aussaat der Fibroblasten in einer Dichte von 20.000 Zellen /cm²
- Kultivierung bei 37 °C, 5 % CO₂ über 6 Tage
- Konditionierter Mediumwechsel an Tag 2 und Tag 5, d.h. die Hälfte des alten Mediums wird entfernt und durch frisches Medium ersetzt
- Zugabe von Na-L-Ascorbat, 50 µg/ml an Tag 1, Tag 2, Tag 5
- Zugabe von Ac₄GalNAz, 50 µM (Endkonzentration) an Tag 0, Tag 2, Tag 5, oder Zugabe von Ac₄GalNAz, 100 µM (Endkonzentration) an Tag 2 und Tag 5
- Lyse der Zellen an Tag 6 durch Waschen mit dest. Wasser und Inkubation in 20 mM NH₄OH (Fig. 2)
- Aufreinigung der FEZM durch Waschen mit destilliertem Wasser

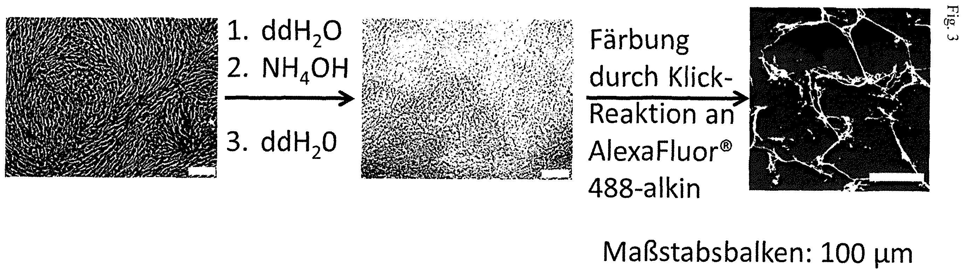

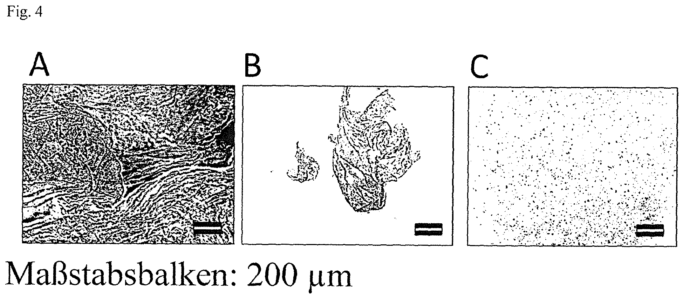

Es konnte gezeigt werden, dass die Fibroblasten bereits nach sechs Tagen eine FEZM ausbilden (Fig. 3). Nach Behandlung mit peracetylierten und azidmodifizierten Monosacchariden wurden die Zellen mit Ammoniumhydroxid lysiert und die FEZM gereinigt. Nach Lyse der Zellen war die FEZM lichtmikroskopisch erkennbar (Figur 3, B). Der Nachweis der Azide in der FEZM erfolgte mittels Fluoreszenzmikroskopie nach Kupplung an einen Alkin-modifizierten Fluorophor (AlexaFluor® 488 alkin) (Figur 3, C). Die nach Kultur aus dermalen Fibroblasten isolierte FEZM liegt zunächst als zusammenhängende Matrix vor (Figur 4, A).

Größere Matrixkomplexe wurden durch sukzessive Zugabe von 0,1%iger Essigsäure (HAc) zur FEZM homogenisiert. Die endgültige Konzentration an Essigsäure sollte 0,1 % betragen. Die FEZM sollte sehr langsam angesäuert werden, da sonst die Gefahr der Denaturierung besteht und die FEZM ausfällt. Daher wurde folgendes Pipettierschema entwickelt, wobei abwechselnd Essigsäure und FEZM zugegeben wird:
- 50 µl HAc (0,01 %)
- + 200 µl FEZM-Suspension, gut mischen
- + 200 µl FEZM-Suspension, gut mischen
- + 50 µl HAc (0,05 %), sehr zügig mischen
- + 200 µl FEZM-Suspension, gut mischen
- + 200 µl FZM-Suspension, gut mischen
- + 200 µl FEZM-Suspension, gut mischen
- + 50 µl HAc (0,05 %), sehr zügig mischen
- + 50 µl HAc (0,1 %), sehr zügig mischen

Die Essigsäure bewirkt, dass größere Strukturen der FEZM aufgebrochen werden, jedoch keine kovalenten Bindungen gespalten werden. Daher kann, je nach Umständen, eine FEZM maximal bis zu bevorzugt 13 Tagen, bevorzugt 10 Tagen, bevorzugt 6 Tagen Zellkultur auf diese Weise in einer Flüssigkeit homogenisiert werden. Nach längerer Zellkultur, bevorzugt nach 14 Tagen, insbesondere bevorzugt nach 21 Tagen, ist dies mit Essigsäure nicht mehr möglich.

Das Homogenisieren größerer Matrixkomplexe in kleinen Einheiten konnte lichtmikroskopisch verfolgt werden, durch Zugabe von Essigsäure (Endkonzentration ∼ 0,1%) wurden alle größeren Komplexe homogenisiert (Figur 4, B+C). Unter dem Lichtmikroskop kann man beobachten, wie aus großen, netzartigen Strukturen kleinere Einheiten entstehen und wie die Suspension homogener in Größe und Form der Bestandteile wird (vgl. Figur 4). Eine Färbung der Proteine mit Fluorescamin ist für eine genauere Betrachtung möglich.

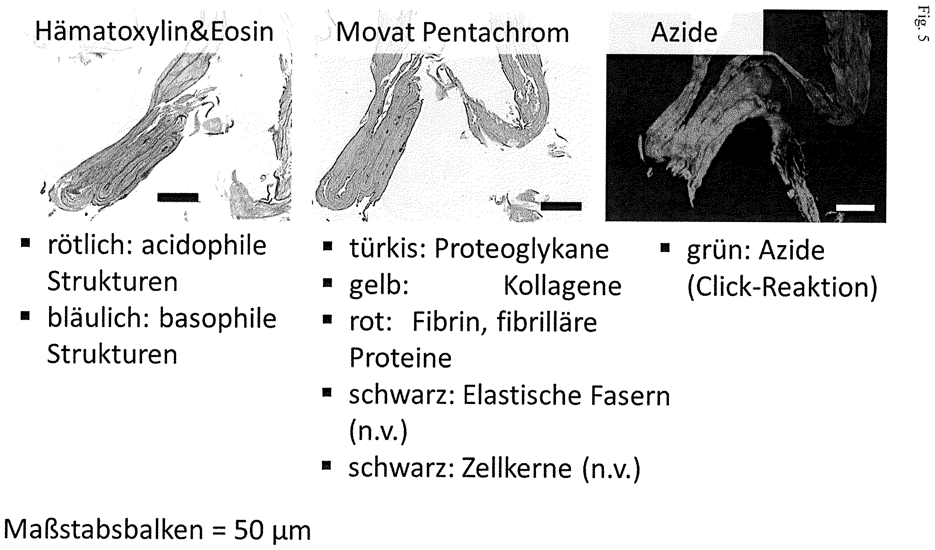

Für die weitere Charakterisierung wurde die FEZM der dermalen Fibroblasten nach 21 Tagen isoliert, in Paraffin eingebettet, und es wurden immunhistochemische Färbungen an Dünnschnitten der FEZM hergestellt. Die Azid-Funktionen wurden in den Paraffin-Dünnschnitten mittels Kupplung an einen Alkin-modifizierten Fluorophor gefärbt. Bei der Hämatoxylin/Eosin-Färbung können basophile Strukturen, wie z.B. die Zellkerne, von acidophilen Strukturen unterschieden werden. Die Movat-Pentachrom-Färbung zeigt, dass die FEZM der dermalen Fibroblasten zu einem großen Anteil aus Proteoglykanen besteht. Daneben sind auch Kollagene und fibrilläre Proteine nachweisbar. Zellkerne oder elastische Fasern konnten in diesem Dünnschnitt nicht mehr nachgewiesen werden (Fig. 5).

Diese Ergebnisse verdeutlichen, dass die Aufreinigung der FEZM erfolgreich war, da keine Zellkerne mehr vorhanden sind. Die Matrix enthält zudem sehr viele Proteoglykane, welche in den Färbungen mit den Azid-Modifikationen colokalisiert sind. Die Azide sind daher in die Proteoglykane der EZM eingebaut.

### Beispiel 3

### Zelladhäsion auf EZM-beschichteten Materialien

Die Adhäsion von Zellen auf EZM-beschichteten Materialien wurde untersucht. Zunächst wurde mit dem Standardprotokoll nachgewiesen, dass EZM beschichtete Substrate die initiale Zelladhäsion epidermaler Keratinozyten verbessern. Bei dem Standardprotokoll werden Zellen auf einem Substrat ausgesät, für 72 h bis zur Konfluenz kultiviert und anschließend lysiert, auf dem Substrat bleibt die EZM als Ablagerung zurück. Titan-besputterte Siliziumwafer (Si-Ti) wurden mit dem Standardprotokoll mit EZM von epidermalen Keratinozyten beschichtet und die Zelladhäsion der Keratinozyten nach 30 Minuten gemessen. Das Zytoskelett der Zellen ist mit Phalloidin-AlexaFluor®546 rot gefärbt, die Zellkerne mit DAPI blau.

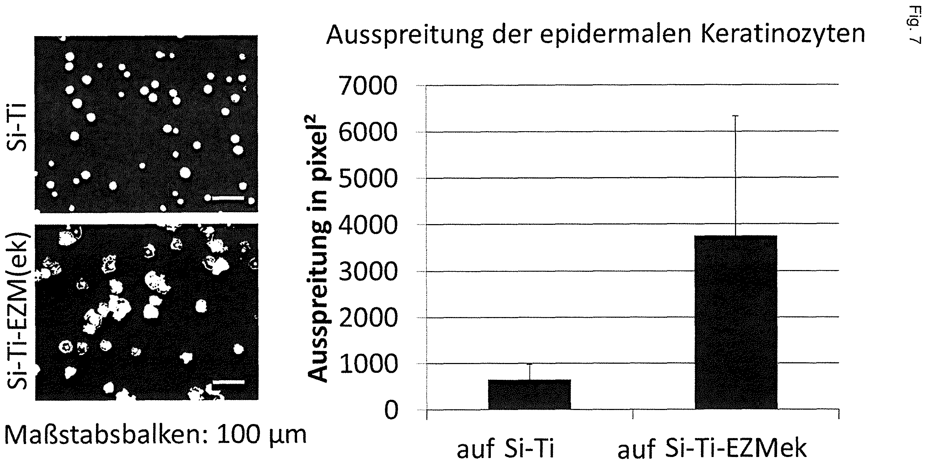

Durch die Beschichtung konnte auf Titan-besputterten Siliziumwafern (Si-Ti) schon nach 30 Minuten Inkubation eine Verbesserung der Zelladhäsion beobachtet werden, die epidermalen Keratinozyten sind auf dem EZM-beschichteten Substrat deutlich stärker ausgespreitet (vgl. Fig. 7).

### Kovalente Bindung einer FEZM auf Biomaterialien

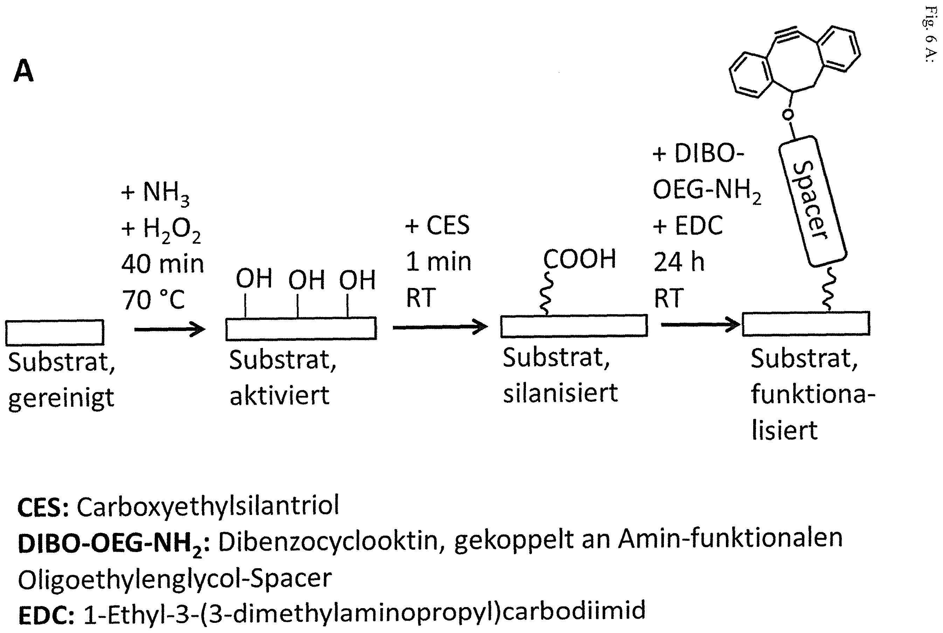

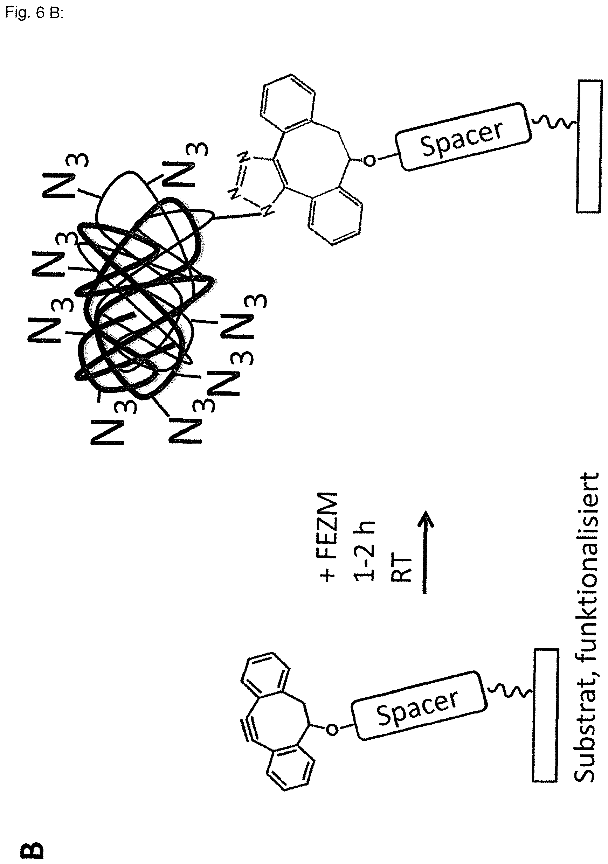

Um eine dauerhafte und stabile Beschichtung von Biomaterialien, dem sogenannten Träger, zu erreichen, wurde das erfindungsgemäße Verfahren entwickelt, mit dem eine FEZM kovalent auf einen Träger beschichtet werden kann (Fig. 6). Für das Anklicken, das heißt kovalente Binden, der Azid-modifizierten EZM aus dermalen Fibroblasten wurde der Träger zunächst nasschemisch mit Dibenzocyclooctin (DIBO) ausgestattet. Der Träger wurde dazu in den folgenden Verfahrensschritten funktionalisiert:
- Aufbringen von Carboxygruppen
   a) durch Silanisierung (zum Beispiel mit Carboxyethylsilantriol, CES) oder
   b) mittels Plasma-Technologie (zum Beispiel Acrylsäureplasma)
- Anknüpfung eines DIBO-Derivats, an das eine Aminogruppe über einen Oligoethylenglycol-Linker angeknüpft ist (DIBO-OEG-NH₂) mittels Carbodiimid-Chemie.

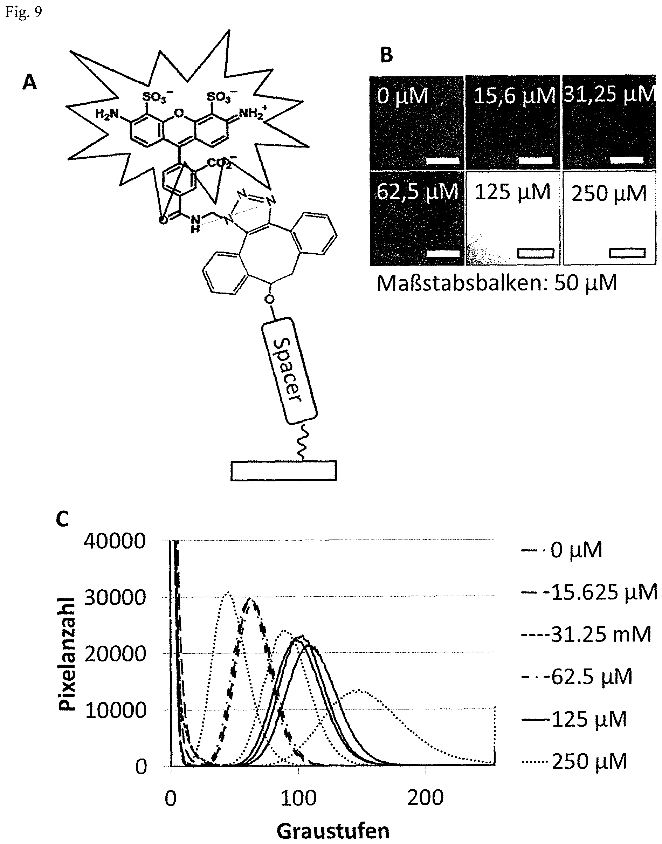

Alternativ können auch Träger, die mit Aktivester-enthaltenden Polymer beschichtet sind (zum Beispiel Nexterion®-Slide H), verwendet werden. Nexterion®-Slide H-Substrate können über die immobilisierten Aktivester schnell und einfach mit DIBO-OEG-NH₂ funktionalisiert werden. Der Nachweis der funktionellen Gruppe DIBO auf der Oberfläche des Trägers erfolgte durch Inkubation mit einem Azidgekoppelten Fluorophor (AlexaFluor®488) (Figur 9, A). Dabei stellte sich heraus, dass eine Konzentration von 125 µM DIBO-OEG-NH₂ für die Funktionalisierung von CES-beschichteten Siliziumwafern geeignet ist. Untersucht wurde dabei die Abhängigkeit der Fluoreszenzintensität von der Konzentration an DIBO-OEG-NH₂ in der Funktionalisierungslösung (0 µM bis 250 µM) (Fig. 9, B). Die Auswertung der Histogramme ergab, dass eine Konzentration von 125 µM DIBO-OEG-NH₂ in der Funktionalisierungslösung die höchste Fluoreszenzintensität ergibt, welche auch über drei Aufnahmen gleichbleibend ist (Fig. 9, C).

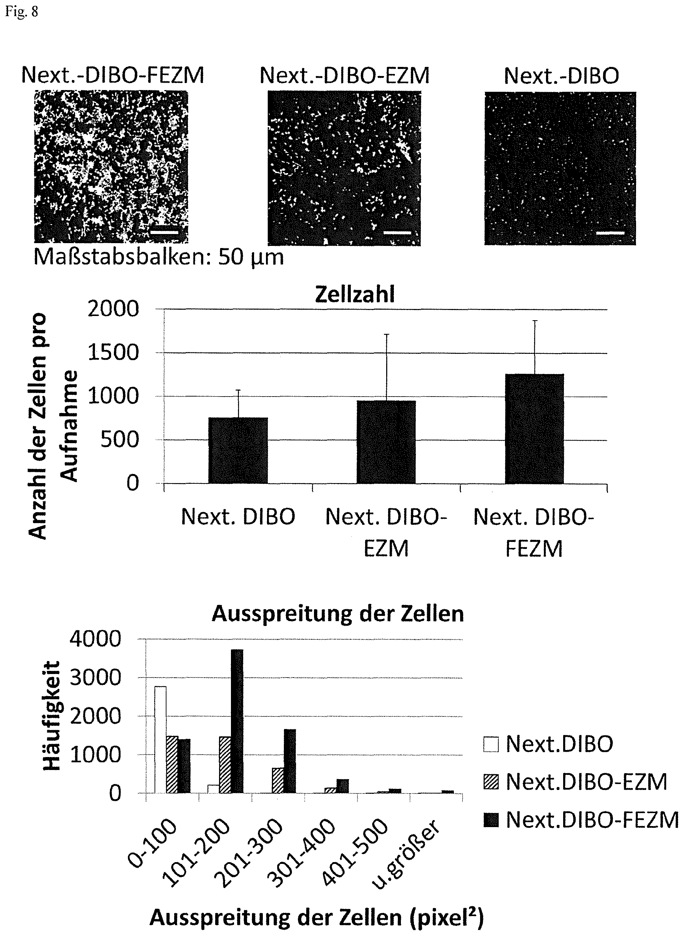

Der gebildete funktionalisierte Träger wurde in einem weiteren Schritt mit homogenisierter FEZM beschichtet, wobei der funktionalisierte Träger ein bis zwei Stunden bei Raumtemperatur mit homogenisierter FEZM, welche eine Proteinkonzentration von 100 µg/ml aufweist, überschichtet wurde. Das Reaktionsschema zur Beschichtung eines Trägers mit FEZM ist in Figur 6 am Beispiel der Silanisierung dargestellt. Die Adhäsion epidermaler Keratinozyten auf FEZM-beschichteten Nexterion®-Slide H-Trägern wurde nach 24 Stunden analysiert (Fig. 8). Als Kontrolle wurde die native EZM verwendet, welche nur über Physisorption auf dem Träger haften kann und nicht kovalent gebunden wird. Das Zytoskelett der Zellen ist mit Phalloidin-AlexaFluor® 546 rot gefärbt, die Zellkerne mit DAPI blau. Die Verbesserung der Zelladhäsion durch FEZM ist anhand der Zellzahl erkennbar; die FEZM-Beschichtung bewirkte eine Erhöhung der Zellzahl. Sehr deutlich ist der Unterschied in Hinblick auf die Ausspreitung der Zellen. Auf dem mit FEZM beschichteten Träger sind die Zellen stärker ausgespreitet als auf dem unbeschichteten oder dem mit nicht-modifizierter EZM beschichteten Träger. Dies liegt daran, dass sich ein Teil der nicht modifizierten EZM beim Waschvorgang wieder abgelöst hat.

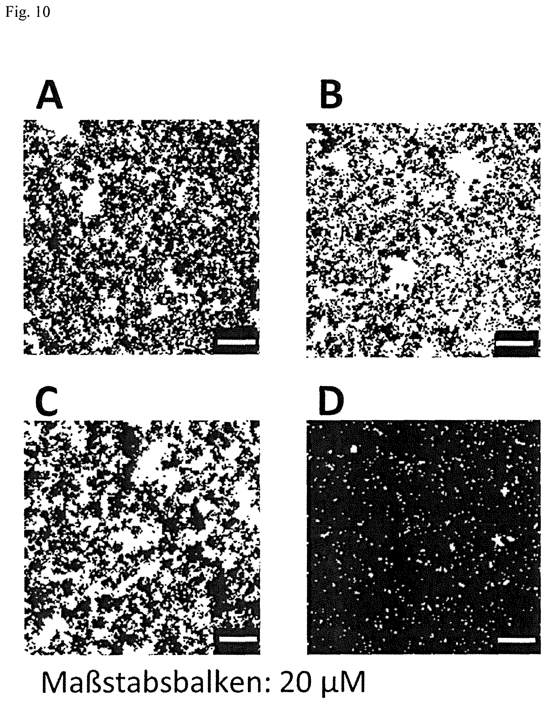

Der Nachweis der FEZM-Beschichtung auf den DIBO-funktionalisierten Siliziumwafern erfolgte mittels Fluoreszenzfärbung mit Fluorescamin. Die Stabilität der Beschichtung wurde durch Waschen der Proben in je 50 ml PBS-Puffer mit 0,1 % Tween 20 (PBST), über Nacht schwenkend, getestet. Die Ergebnisse der Fluoreszenzfärbung sind in Figur 10 dargestellt, dabei wurde je ein mit DIBO funktionalisierter Siliziumwafer entweder mit FEZM (Fig. 10; A, B) oder mit nicht-modifizierter EZM (Fig. 10; C, D) beschichtet. Dieses Ergebnis zeigt, dass nur die kovalent gekoppelte FEZM auch nach dem intensiven Waschen noch als Beschichtung vorhanden ist (Fig. 10; C), während die physisorbierte nicht-modifizierte EZM abgewaschen wird (Fig. 10, D). Die kovalente Beschichtung mit der FEZM ist damit wesentlich stabiler als eine physisorbierte Beschichtung.

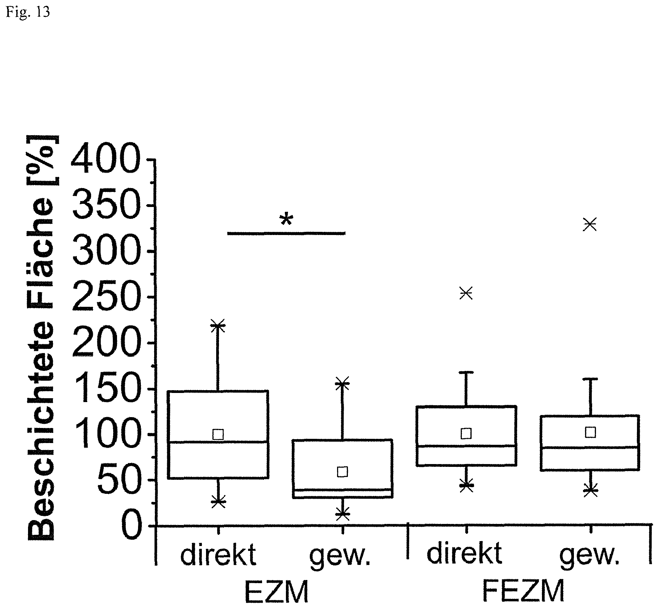

Die Stabilität der Beschichtung mit einer EZM im Vergleich mit einer FEZM wurde auch durch die Quantifizierung der prozentualen beschichteten Fläche direkt nach dem Aufbringen der Beschichtung auf silanisierte und DIBO-funktionalisierte Siliziumwafer (direkt) bzw. auf intensiv gewaschenen Beschichtungen ("gew.") ermittelt (Fig. 13). Für die Vergleichbarkeit zwischen einzelnen Versuchen erfolgte eine Normierung der Werte, wobei der Mittelwert der "direkten" Werte jeweils als 100 % definiert wurde. Der Versuch wurde mit EZMs von drei unterschiedlichen Spendern wiederholt (n=3), ein statistisch signifikanter Unterschied zwischen FEZM und EZM war bei einem Signifikanzniveau von α = 0,05 messbar (ANOVA mit Post Hoc Test nach Bonferroni). Die beschichtete Fläche einer intensiv gewaschenen Beschichtung aus FEZM ist signifikant höher im Vergleich zu einer Beschichtung aus EZM. Die FEZM wird von dem Träger also deutlich weniger leicht abgewaschen und bindet signifikant stärker an diesen.

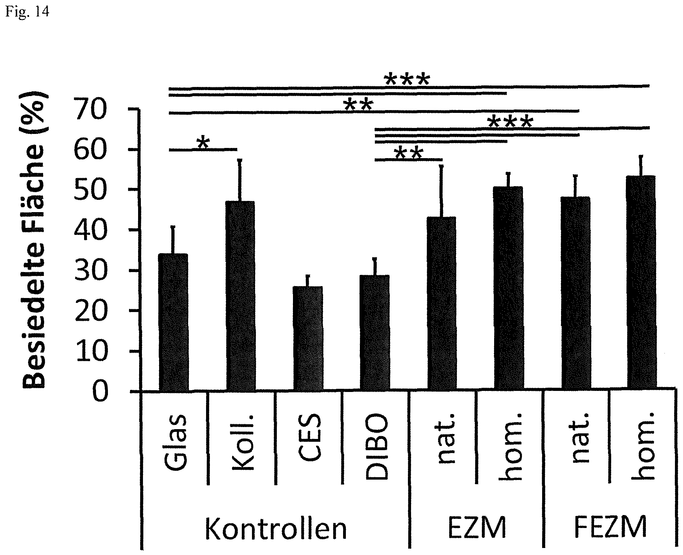

Das Zellwachstum von Zellen der Keratinozytenzellinie HaCaT (human, adult, high calcium, low temperature) auf FEZM-beschichteten Glassubstraten wurde untersucht. Es wurden Beschichtungen mit nativer und homogenisierter FEZM und nativer und homogenisierter EZM und den Kontrollen Glas, Kollagen, silanisiertem Glas (CES) und DIBO-funktionalisiertem Glas verglichen. Die Quantifizierung erfolgte nach vier Tagen durch Messung der mit Zellen besiedelten Fläche (Fig. 14), Signifikanzniveau α = 0,05 (*), α = 0,01 (**) bzw. α = 0,001 (***) (ANOVA mit Post Hoc Test nach Bonferroni).

Es zeigt sich, dass eine Beschichtung mit FEZM das Wachstum der HaCaT-Zellen deutlich (signifikant) erhöht, verglichen mit unbeschichteten Oberflächen. Den gleichen Effekt bewirkt auch die EZM-Beschichtung. Dies zeigt, dass die funktionelle Gruppe A (hier: die Azide) das Zellwachstum nicht unerwünscht beeinflusst. Darüber hinaus zeigt sich, dass sowohl Beschichtungen mit homogenisierter als auch mit nativer FEZM für die Beschichtung geeignet sind, da sie den gleichen positiven (d.h. Zellwachstums-fördernden) Effekt auf die HaCaT-Zellen bewirken.

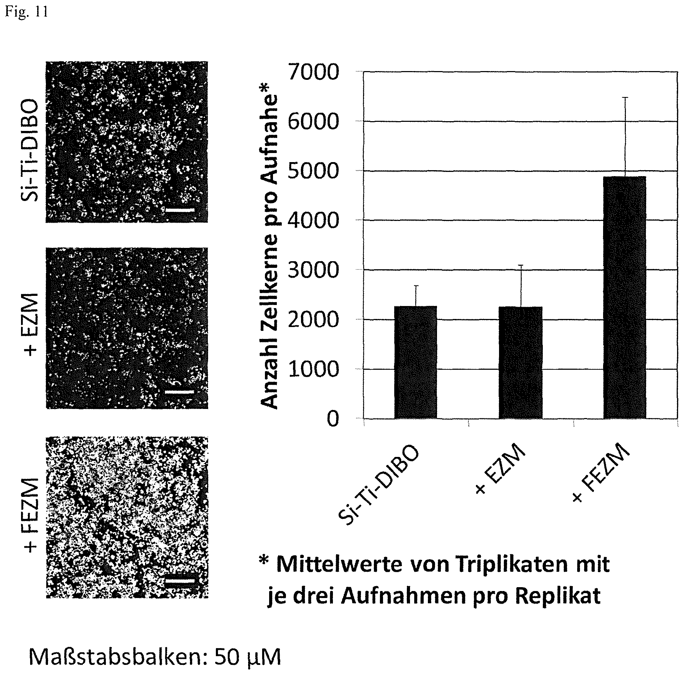

Für die FEZM-Beschichtung anderer Materialien ist zunächst eine Modifizierung der Oberflächen mit einer komplementären funktionellen Gruppe notwendig. Diese wurde auf Siliziumwafern bzw. auf Titan-besputterten Siliziumwafern etabliert. Die Wafer wurden dazu nasschemisch mit Carboxyethylsilantriol silanisiert und mit DIBO-OEG-NH₂ funktionalisiert. Die Beschichtung mit FEZM erfolgte durch kupferfreie Kupplung an eine DIBO-modifizierte Oberfläche. Als Kontrolle wurden die Wafer mit nicht-modifizierter EZM beschichtet. Das Zytoskelett der Zellen wurde anschließend mit Phalloidin-AlexaFluor®546 rot gefärbt, die Zellkerne wurden mit DAPI blau gefärbt. Auf den FEZM-beschichteten Titansubstraten ist eine Verbesserung der Adhäsion von Keratinozyten zu beobachten (Fig. 11), die Zellzahl auf den mit FEZM beschichteten Substraten ist deutlich erhöht. Dagegen bewirkt eine Beschichtung mit der nicht-modifizierten EZM keine Veränderung der Zelladhäsion. Die Beschichtung über eine kovalente Kupplung der FEZM auf die funktionalisierte Titan-Oberfläche ist deutlich stabiler.

### Beispiel 4

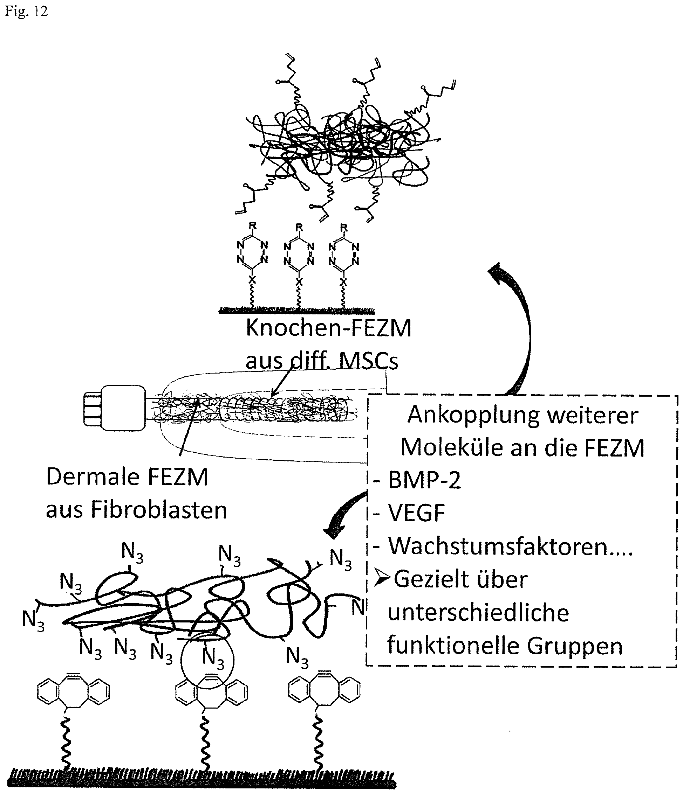

Die gemäß der vorliegenden Erfindung beschriebene FEZM kann zur Beschichtung von Implantaten eingesetzt werden. Dargestellt ist der prinzipielle Aufbau einer Endo-Exo-Prothese, welche bei Beinamputierten Patienten Verwendung findet (vgl. Figur 12). Dabei wird eine Titanprothese im Femurknochen des Patienten verschraubt. Über einen perkutanen Austritt kann extern eine Beinprothese angebracht werden. Hierbei kann die aus dermalen Fibroblasten gewonnene FEZM am perkutanen Austritt angekoppelt werden, während für die Titanschraube eine knochenähnliche Matrix aus mesenchymalen Stammzellen geeignet ist. Die Oberflächeneigenschaften des Implantats werden dadurch verbessert, insbesondere führt die Beschichtung mit FEZM zu einer besseren Gewebeeinheilung und verbessert die Belastbarkeit, Funktion und Lebensdauer und führt auch zu einer besseren Wundheilung. Des Weiteren können zusätzlich Biomoleküle an die FEZM angekoppelt werden, um das Einwachsen des Implantats zu unterstützen.

## Patentansprüche

1. Eine funktionalisierte extrazelluläre Matrix (FEZM) umfassend Proteoglykane und Glykoproteine, die mindestens eine funktionelle Gruppe A aufweist, wobei die mindestens eine funktionelle Gruppe A kovalent in Oligosaccharidketten der Proteoglykane oder Glykoproteine eingebaut ist, wobei die mindestens eine funktionelle Gruppe A fähig ist, mindestens eine kovalente Bindung mit einer komplementären funktionellen Gruppe B einzugehen und wobei die funktionelle Gruppe A ausgewählt ist aus der Gruppe bestehend aus einer Azidgruppe, C-C-Doppelbindung und C-C-Dreifachbindung.

2. Die funktionalisierte extrazelluläre Matrix nach Anspruch 1, wobei die mindestens eine funktionelle Gruppe A eine Azidgruppe ist.

3. Die funktionalisierte extrazelluläre Matrix nach einem der Ansprüche 1 und 2, wobei die funktionelle Gruppe B ausgewählt ist aus der Gruppe bestehend aus einer Azidgruppe, C-C-Doppelbindung, C-C-Dreifachbindung, Nitrilimingruppe, Nitriloxidgruppe, Phosphingruppe, Phosphitgruppe und Tetrazingruppe.

4. Verfahren zur in-vitro-Herstellung einer funktionalisierten extrazellulären Matrix nach einem der Ansprüche 1 bis 3, umfassend die folgenden Verfahrensschritte:
a) Bereitstellen von Zellen,
b) Behandeln und Kultivieren der Zellen mit mindestens einem chemischen Agens enthaltend eine funktionelle Gruppe A und wobei die funktionelle Gruppe A ausgewählt ist aus der Gruppe bestehend aus einer Azidgruppe, C-C-Doppelbindung und C-C-Dreifachbindung unter Bedingungen, dass eine funktionalisierte extrazelluläre Matrix erzeugt wird, und
c) Gewinnen der funktionalisierten extrazellulären Matrix.

5. Verfahren nach Anspruch 4, wobei die bereitgestellten Zellen humane oder tierische Zellen sind.

6. Verfahren nach einem der Ansprüche 4 oder 5, wobei die bereitgestellten Zellen ausgewählt sind aus der Gruppe bestehend aus Epithelzellen, Endothelzellen, Fibroblasten, Stammzellen sowie Zellen des Binde- und Stützgewebes.

7. Verfahren nach einem der Ansprüche 4 bis 6, wobei das chemische Agens ein funktionalisiertes Saccharid mit mindestens einer funktionellen Gruppe A ist, bevorzugt ein peracyliertes Saccharid, insbesondere ein Mono- oder Disaccharid.

8. Verfahren nach einem der Ansprüche 4 bis 7, wobei im Verfahrensschritt b) Natrium-L-ascorbat hinzugegeben wird.

9. Verfahren nach einem der Ansprüche 4 bis 8, wobei die Zellen nach Verfahrensschritt b) und vor Verfahrensschritt c) entfernt werden.

10. Funktionalisierte extrazelluläre Matrix, insbesondere nach einem der Ansprüche 1 bis 3, herstellbar nach einem der Verfahren der Ansprüche 4 bis 9.

11. Ein FEZM-Träger-Verbund, umfassend eine FEZM nach einem der Ansprüche 1 bis 3 oder 10 und einen funktionalisierten Träger, wobei der funktionalisierte Träger mindestens eine funktionelle Gruppe B aufweist, die fähig ist, mit mindestens einer funktionellen Gruppe A einer funktionalisierten extrazellulären Matrix mindestens eine kovalente Bindung auszubilden, wobei die funktionelle Gruppe B ausgewählt ist aus der Gruppe bestehend aus einer Azidgruppe, C-C-Doppelbindung, C-C-Dreifachbindung, Nitrilimingruppe, Nitriloxidgruppe, Phosphingruppe, Phosphitgruppe und Tetrazingruppe, wobei die FEZM durch mindestens eine von der mindestens einen funktionellen Gruppe A mit der mindestens einen komplementären funktionellen Gruppe B gebildete kovalente Bindung an die Oberfläche des Trägers gebunden ist, wobei gilt:
i) wenn die funktionelle Gruppe A eine Azidgruppe ist, ist die funktionelle Gruppe B eine C-C-Doppelbindung, C-C-Dreifachbindung, C-N-Dreifachbindung, Phosphingruppe oder Phosphitgruppe,
ii) wenn die funktionelle Gruppe A eine C-C-Doppelbindung ist, ist die funktionelle Gruppe B eine Azidgruppe, C-C-Doppelbindung, C-C-Dreifachbindung Nitrilimingruppe, Nitriloxidgruppe oder Tetrazingruppe und
iii) wenn die funktionelle Gruppe A eine C-C-Dreifachbindung ist, ist die funktionelle Gruppe B eine Azidgruppe, C-C-Doppelbindung, Nitriloxidgruppe oder Tetrazingruppe.

12. Der FEZM-Träger-Verbund nach Anspruch 11, wobei der funktionalisierte Träger ausgewählt ist aus der Gruppe bestehend aus Kunststoff, Keramik, Glas, Silizium, Kohlenstoff oder einem Metall.

13. Der FEZM-Träger-Verbund nach einem der Ansprüche 11 oder 12, wobei der Träger ein Zellkulturgefäß, ein Implantat oder eine Prothese ist.

14. Der FEZM-Träger-Verbund nach einem der Ansprüche 11 bis 13, wobei die funktionelle Gruppe B Cyclooctin oder ein Derivat davon ist.

15. Der FEZM-Träger-Verbund nach einem der Ansprüche 11 bis 14, wobei die mindestens eine kovalente Bindung zwischen einer Azidgruppe als funktionelle Gruppe A mit Cyclooctin oder einem Derivat davon als funktionelle Gruppe B gebildet ist.

16. Verfahren zur Herstellung eines FEZM-Träger-Verbunds nach einem der Ansprüche 11 bis 15, wobei eine funktionalisierte extrazelluläre Matrix mit mindestens einer funktionellen Gruppe A nach einem der Ansprüche 1 bis 3 mit einem funktionalisierten Träger, wobei der funktionalisierte Träger mindestens eine funktionelle Gruppe B aufweist, die fähig ist, mit mindestens einer funktionellen Gruppe A einer funktionalisierten extrazellulären Matrix mindestens eine kovalente Bindung auszubilden, wobei die funktionelle Gruppe B ausgewählt ist aus der Gruppe bestehend aus einer Azidgruppe, C-C-Doppelbindung, C-C-Dreifachbindung, Nitrilimingruppe, Nitriloxidgruppe, Phosphingruppe, Phosphitgruppe und Tetrazingruppe, unter Bedingungen umgesetzt wird, dass die funktionalisierte extrazelluläre Matrix über mindestens eine kovalente Bindung zwischen der funktionellen Gruppe A und B auf dem funktionalisierten Träger gebunden wird.

17. Verwendung des FEZM-Träger-Verbunds nach einem der Ansprüche 11 bis 15 oder hergestellt nach einem Verfahren nach Anspruch 16 zur Modifizierung der Oberflächen von Biomaterialien, Medizinprodukten und Zellkultursubstraten.

## Claims

1. Functionalised extracellular matrix (FECM) comprising proteoglycans and glycoproteins, which has at least one functional group A, wherein the at least one functional group A is covalently incorporated into oligosaccharide chains of the proteoglycans or glycoproteins, wherein the at least one functional group A is capable of forming at least one covalent bond with a complementary functional group B, and wherein the functional group A is selected from the group consisting of an azide group, C-C double bond and C-C triple bond.

2. Functionalised extracellular matrix according to claim 1, wherein the at least one functional group A is an azide group.

3. Functionalised extracellular matrix according to claim 1 or claim 2, wherein the functional group B is selected from the group consisting of an azide group, C-C double bond, C-C triple bond, nitrile imine group, nitrile oxide group, phosphine group, phosphite group and tetrazine group.

4. Process for in-vitro-production of a functionalised extracellular matrix according to anyone of claims 1 to 3, comprising the following process steps:
a) providing cells,
b) treating and cultivating the cells using at least one chemical agent including a functional group A and wherein the functional group A is selected from the group consisting of an azide group, C-C double bond and C-C triple bond, under conditions such that a functionalised extracellular matrix is generated, and
c) obtaining the functionalised extracellular matrix.

5. Process according to claim 4, wherein the provided cells are human or animal cells.

6. Process according to claim 4 or claim 5, wherein the provided cells are selected from the group consisting of epithelial cells, endothelial cells, fibroblasts, stem cells, and cells of the connective and supporting tissue.

7. Process according to anyone of claims 4 to 6, wherein the chemical agent is a functionalised saccharide with at least one functional group A, preferably a peracylated saccharide, in particular a mono- or disaccharide.

8. Process according to anyone of claims 4 to 7, wherein sodium L-ascorbate is added in process step b).

9. Process according to anyone of claims 4 to 8, wherein the cells are removed after process step b) and before process step c).

10. Functionalised extracellular matrix, in particular according to anyone of claims 1 to 3, producible by anyone of the processes of claims 4 to 9.

11. FECM-carrier-compound, comprising an FECM according to anyone of claims 1 to 3 or 10 and a functionalised carrier, wherein the functionalised carrier has at least one functional group B, which is capable of forming a covalent bond with at least one functional group A of a functionalised extracellular matrix, wherein the functional group B is selected from the group consisting of an azide group, C-C double bond, C-C triple bond, nitrile imine group, nitrile oxide group, phosphine group, phosphite group and tetrazine group, wherein the FECM is bonded to the surface of the carrier by at least one covalent bond, which is formed by at least one of the at least one functional group A with the at least one complementary functional group B, wherein:
i) if the functional group A is an azide group, the functional group B is a C-C double bond, C-C triple bond, C-N triple bond, phosphine group or phosphite group,
ii) if the functional group A is a C-C double bond, the functional group B is an azide group, C-C double bond, C-C triple bond, nitrile imine group, nitrile oxide group or tetrazine group, and
iii) if the functional group A is a C-C triple bond, the functional group B is an azide group, C-C double bond, nitrile oxide group or tetrazine group.

12. FECM-carrier-compound according to claim 11, wherein the functionalised carrier is selected from the group consisting of plastics material, ceramic, glass, silicon, carbon or a metal.

13. FECM-carrier-compound according to claim 11 or claim 12, wherein the carrier is a cell culture vessel, an implant or a prosthesis.

14. FECM-carrier-compound according to anyone of claims 11 to 13, wherein the functional group B is cyclooctyne or a derivative thereof.

15. FECM-carrier-compound according to anyone of claims 11 to 14, wherein the at least one covalent bond between the azide group as the functional group A is formed, with cyclooctyne or a derivative thereof as the functional group B.

16. Process for producing an FECM-carrier-compound according to anyone of claims 11 to 15, wherein a functionalised extracellular matrix with at least one functional group A according to anyone of claims 1 to 3 is reacted with a functionalised carrier, wherein the functionalised carrier has at least one functional group B which is capable of forming at least one covalent bond with at least one functional group A of a functionalised extracellular matrix, wherein the functional group B is selected from the group consisting of an azide group, C-C double bond, C-C triple bond, nitrile imine group, nitrile oxide group, phosphine group, phosphite group and tetrazine group, under conditions such that the functionalised extracellular matrix is bonded via at least one covalent bond between the functional groups A and B on the functionalised carrier.

17. Use of the FECM-carrier-compound according to anyone of claims 11 to 15 or produced by a process according to claim 16 for modifying the surfaces of biomaterials, medicinal products and cell culture substrates.

## Revendications

1. Matrice extracellulaire fonctionnalisée (FEZM), comprenant des protéoglycanes et des glycoprotéines, qui présente au moins un groupe fonctionnel A, dans laquelle l'au moins un groupe fonctionnel A est intégré de manière covalente dans des chaînes oligosaccharides des protéoglycanes ou des glycoprotéines, dans laquelle l'au moins un groupe fonctionnel A est capable d'être impliqué dans au moins une liaison covalente avec un groupe fonctionnel B complémentaire et dans laquelle le groupe fonctionnel A est choisi dans le groupe constitué d'un groupement azoture, d'une liaison double C-C et d'une liaison triple C-C.

2. Matrice extracellulaire fonctionnalisée selon la revendication 1, dans laquelle l'au moins un groupe fonctionnel A est un groupement azoture.

3. Matrice extracellulaire fonctionnalisée selon l'une des revendications 1 et 2, dans laquelle le groupe fonctionnel B est choisi dans le groupe constitué d'un groupement azoture, d'une liaison double C-C, d'une liaison triple C-C, d'un groupement nitrilimine, d'un groupement oxyde de nitrile, d'un groupement phosphine, d'un groupement phosphite et d'un groupement tétrazine.

4. Procédé de fabrication in-vitro d'une matrice extracellulaire fonctionnalisée selon l'une des revendications 1 à 3, comprenant les étapes de procédé suivantes :
a) la préparation de cellules,
b) le traitement et la culture des cellules avec au moins un agent chimique contenant un groupe fonctionnel A et dans laquelle le groupe fonctionnel A est choisi dans le groupe constitué d'un groupement azoture, d'une liaison double C-C et d'une liaison triple C-C, à condition qu'une matrice extracellulaire fonctionnalisée se trouve créée, et
c) l'obtention de la matrice extracellulaire fonctionnalisée.

5. Procédé selon la revendication 4, dans lequel les cellules préparées sont des cellules humaines ou animales.

6. Procédé selon l'une des revendications 4 ou 5, dans lequel les cellules préparées sont choisies dans le groupe constitué des cellules épithéliales, des cellules endothéliales, des fibroblastes, des cellules souches telles que des cellules de tissu conjonctif et de tissu de support.

7. Procédé selon l'une des revendications 4 à 6, dans lequel l'agent chimique est un saccharide fonctionnalisé avec au moins un groupe fonctionnel A, de préférence, un saccharide peracylé, notamment, un mono ou un di saccharide.

8. Procédé selon l'une des revendications 4 à 7, dans lequel on ajoute un L-ascorbate de sodium dans l'étape de procédé b).

9. Procédé selon l'une des revendications 4 à 8, dans lequel les cellules sont enlevées après l'étape de procédé b) et avant l'étape de procédé c).

10. Matrice extracellulaire fonctionnalisée, notamment selon l'une des revendications 1 à 3, pouvant être fabriquée d'après un procédé selon les revendications 4 à 9.

11. Composite FEZM-support, comprenant une FEZM selon l'une des revendications 1 à 3 ou 10 et un support fonctionnalisé, dans lequel le support fonctionnalisé présente au moins un groupe fonctionnel B qui est capable de former au moins une liaison covalente avec au moins un groupe fonctionnel A d'une matrice extracellulaire fonctionnalisée, dans lequel le groupe fonctionnel B est choisi dans un groupe constitué d'un groupement azoture, d'une liaison double C-C, d'une liaison triple C-C, d'un groupement nitrilimine, d'un groupement oxyde de nitrile, d'un groupement phosphine, d'un groupement phosphite et d'un groupement tétrazine, dans lequel la FEZM est reliée à la surface du support par au moins une liaison covalente formée par l'au moins un groupe fonctionnel A avec l'au moins un groupe fonctionnel B complémentaire, dans lequel :
i) lorsque le groupe fonctionnel A est un groupement azoture, le groupe fonctionnel B est une double liaison C-C, une triple liaison C-C, une triple liaison C-N, un groupement phosphine ou un groupement phosphite,
ii) lorsque le groupe fonctionnel A est une double liaison C-C, le groupe fonctionnel B est un groupement azoture, une double liaison C-C, une triple liaison C-C, un groupement nitrilimine, un groupement oxyde de nitrile ou un groupement tétrazine, et
iii) lorsque le groupe fonctionnel A est une triple liaison C-C, le groupe fonctionnel B est un groupement azoture, une double liaison C-C, un groupement oxyde de nitrile ou un groupement tétrazine.

12. Composite FEZM-support selon la revendication 11, dans lequel le support fonctionnalisé est choisi dans le groupe constitué d'une matière plastique, d'une céramique, de verre, de silicium, de carbone ou d'un métal.

13. Composite FEZM-support selon l'une des revendications 11 ou 12, dans lequel le support est un récipient de culture cellulaire, un implant ou une prothèse.

14. Composite FEZM-support selon l'une des revendications 11 à 13, dans lequel le groupe fonctionnel B est de la cyclooctyne ou un de ses dérivés.

15. Composite FEZM-support selon l'une des revendications 11 à 14, dans lequel l'au moins une liaison covalente est formée entre un groupement azoture servant de groupe fonctionnel A avec de la cyclooctyne ou un de ses dérivés servant de groupe fonctionnel B.

16. Procédé de fabrication d'un composite FEZM-support selon l'une des revendications 11 à 15, dans lequel une matrice extracellulaire fonctionnalisée avec au moins un groupe fonctionnalisé A selon l'une des revendications 1 à 3 est mise à réagir avec un support fonctionnalisé, dans lequel le support fonctionnalisé présente au moins un groupe fonctionnel B qui est capable, avec au moins un groupe fonctionnel A d'une matrice extracellulaire fonctionnalisée, de former une liaison covalente, dans lequel le groupe fonctionnel B est choisi dans le groupe constitué d'un groupement azoture, d'une double liaison C-C, d'une triple liaison C-C, d'un groupement nitrilimine, d'un groupement oxyde de nitrile, d'un groupement phosphine, d'un groupement phosphite et d'un groupement tétrazine, à condition que la matrice extracellulaire fonctionnalisée est reliée sur le support fonctionnalisé par le biais d'au moins une liaison covalente entre les groupes fonctionnels A et B.

17. Utilisation du composite FEZM-support selon l'une des revendications 11 à 15 ou fabriqué d'après un procédé selon la revendication 16 pour la modification des surfaces de biomatériaux, de produits médicaux et de substrats de culture cellulaire.
